# FASCICULE DE BREVET EUROPEEN

(11) **EP 2 475 315 B1**
(45) Date de publication et mention de la délivrance du brevet: **07.02.2018**
(21) Numéro de dépôt: 10763201.0
(22) Date de dépôt: 07.09.2010
(51) Int. Cl.: A61B 17/72, A61B 17/80, A61F 2/30

(54) **DISPOSITIF INTRA-CORPOREL POUR LE DEPLACEMENT DE TISSUS**
INTRAKORPORALE VORRICHTUNG ZUR GEWEBEBEWEGUNG
INTRACORPOREAL DEVICE FOR MOVING TISSUE

(30) Priorité: 09.09.2009 FR 0904306
(43) Date de publication de la demande: 18.07.2012
(73) Titulaire: Soubeiran, Alicia Eloina, 75014 Paris (FR); Soubeiran, Marine Alicia, 75014 Paris (FR); Soubeiran, Adrien André, 75014 Paris (FR); Soubeiran, Constance Eloina, 75014 Paris (FR)
(72) Inventeur: SOUBEIRAN, Arnaud, décédé (FR)
(74) Mandataire: Demulsant, Xavier
(86) Numéro de dépôt international: PCT/FR2010/000608
(87) Numéro de publication internationale: WO 2011/030015

(56) Documents cités:
- WO-A2-2007/144489
- FR-A1- 2 267 080
- FR-A1- 2 906 453
- FR-A1- 2 916 622

## Description

### Domaine Technique

L'invention se rapporte à un dispositif pour le déplacement des tissus dans le corps humain ou animal.

L'invention concerne plus particulièrement, mais non exclusivement, les dispositifs permettant de déplacer des portions d'os.

L'invention trouve notamment une application avantageuse dans les secteurs techniques suivants :
- tiges de distraction ou de compression pour la correction du rachis ou du thorax en prenant appui sur des vertèbres, des côtes et/ou le bassin;
- clou médullaire ou plaque d'allongement ou de transport osseux pour les os longs, plats, de la mâchoire ou du crâne;
- prothèses de croissance.

### Technique Antérieure

L'allongement osseux par distraction progressive du cal qui se forme naturellement quand un os est fracturé avant qu'il ne se calcifie a été initialement réalisé à l'aide de fixateurs externes, par exemple tel que popularisé par Ilizarov.

Pour tenter de corriger les inconvénients des fixateurs externes, tels que les infections ou la gêne pour la vie quotidienne, des clous d'allongement totalement implantés ont été proposés : clou Albizzia pour lequel l'allongement est provoqué par une rotation volontaire de la jambe du patient, clou de Bliskunov, clou de Baumgart et Betz, ou encore le clou ISKD (*Intramedullary Skeletal Kinetic Distractor*) de Cole.

La vitesse d'allongement de l'os, de l'ordre d'un millimètre par jour, est modulée en fonction des observations cliniques.

Le transport osseux permet de reconstruire, par exemple, une partie de la diaphyse d'un os supprimée suite à un traumatisme, une infection ou une tumeur, en étirant progressivement le cal qui se forme entre une portion d'os préservée et une rondelle d'os que l'on a détachée par ostéotomie de cette portion, jusqu'à ce que la rondelle vienne en butée contre la seconde portion d'os préservée.

Les documents WO 02/071962 et WO 95/24870 présentent des exemples de clous de transports osseux. Ces deux dispositifs fonctionnent par des systèmes à cliquet, se révèlent encombrants, comportent un grand nombre de pièces, et sont difficilement miniaturisables. Les mouvements nécessaires à leur allongement sont fréquemment douloureux.

La distraction par des moyens intracorporels pour le traitement chirurgical de déformations du rachis a également été proposée, en particulier pour le traitement de scolioses évolutives chez l'enfant. Une présentation fonctionnelle des instrumentations de traitement chirurgical des déformations du rachis est donnée par Foster et al (The Spine Journal, pp. 652-694, 2005)*.*

Chez l'enfant, il faut non seulement corriger au mieux la déformation, mais aussi maintenir la correction obtenue tout au long de la croissance en limitant le moins possible cette dernière. Ceci nécessite que la géométrie du distracteur implanté évolue au cours du temps. Pour ce faire, la plupart des distracteurs connus nécessitent des chirurgies répétées, avec les difficultés, les coûts et les risques associés, notamment infectieux. C'est le cas des dispositifs décrits dans les documents WO 2006/010844, WO 2007/051924, FR 2900563, FR 2843538, FR 2891727, FR 2794357 et FR 2892617. C'est également le cas du VEPTR ® (*Vertical Expandable Prosthetic Titanium Rib*). Afin de tenter de limiter le nombre d'opérations chirurgicales, plusieurs dispositifs intracorporels pouvant être allongés sans réopération ont été proposés. Parmi exemple, le distracteur rachidien décrit dans le document WO 01/78614 comprend un aimant pilotant en rotation une roue engrenant deux roues diamétralement opposées et pourvues chacune d'un taraudage complémentaire du filetage de deux tiges d'élongation.

L'utilisation d'engrenages et de filetages sur les tiges d'élongation nécessite de rendre le dispositif étanche aux matières biologiques environnantes, ce qui est techniquement très délicat et limite les possibilités de stérilisation. En outre, la présence d'un filetage sur les tiges d'élongation qui sont très sollicitées en fatigue limite la durée de vie de l'implant ou impose son sur-dimensionnement, qui le rend alors trop volumineux pour être raisonnablement implanté chez un enfant.

Le document WO 2007/144489 au nom du demandeur décrit un dispositif d'allongement intracorporel, ce dispositif antérieur trouvant application en tant que clou d'allongement osseux, clou de transport osseux, tige de distraction rachidienne ou prothèse de croissance. Le dispositif décrit dans le document WO 2007/144489 comprend :
- une première partie allongée,
- une seconde partie, montée télescopique par rapport à la première partie,
- des premiers moyens de liaison à l'organisme, par exemple par vissage, à une première extrémité de la première partie,
- des seconds moyens de liaison à l'organisme, par exemple par vissage, à une première extrémité de la seconde partie,
- une tige comportant au moins un filetage dont la rotation entraîne le déplacement de la seconde partie par rapport à la première partie,
- des moyens pour commander la rotation de la tige, par exemple un aimant permanent, ladite tige étant montée entre deux extrémités qui se rapprochent lorsque le dispositif s'allonge.

Dans le mode de réalisation préféré décrit dans ce document antérieur du demandeur, la tige comprend, à une première partie extrême, un logement pour un aimant permanent. Une languette d'appui est fixée, par exemple par soudage, à la seconde partie extrême de la tige. Cette languette d'appui est fixée à la première partie tubulaire du dispositif, par exemple par soudage, et est guidée en coulissement longitudinal par rapport à la deuxième partie du dispositif. Entre le logement de l'aimant permanent et la languette d'appui, la tige filetée est vissée dans un taraudage de la deuxième partie du dispositif. Une longueur de tige chargée est ainsi définie entre le taraudage de la deuxième pièce et la pièce d'appui solidaire de la première pièce du dispositif.

Ce dispositif antérieur présente de nombreux avantages. En particulier, il est simple et peu couteux à fabriquer, au regard de la fonction remplie, présente une puissance suffisante à sa fonction dans un volume réduit, ne nécessite pas d'étanchéité particulière, peut être commandé manuellement et sans douleur à l'aide d'un simple aimant permanent externe, notamment à domicile, par le patient ou une personne qui l'assiste sans qualification particulière. En outre, la longueur de la tige chargée diminue lorsque le dispositif s'allonge et la longueur de tige chargée travaille en traction quand le dispositif s'allonge, ce qui prévient toute possibilité de flambage.

Le dispositif décrit dans le document WO 2007/144489 présente toutefois, pour la puissance et le potentiel d'allongement souhaités, une longueur droite rigide qui le rend impossible à placer dans une localisation qui est toute en courbes (comme dans les applications au rachis et au thorax par exemple), ou dans une localisation où l'espace manque en longueur dans la direction du déplacement que l'on souhaite réaliser graduellement (comme pour les transports ou allongements osseux importants, ou ceux de la mâchoire par exemple). En outre ce dispositif est apte à exercer une distraction ou une compression, mais pas indifféremment les deux, car il se désassemble si un effort est appliqué dans la direction opposée à celle de l'effort qu'il est capable de produire. L'invention vise notamment à lever ces limites, tout en conservant les avantages du dispositif décrit dans le document WO 2007/144489, en proposant un nouveau dispositif intracorporel d'allongement à vis, à longueur réglable, notamment, mais non exclusivement, pour une application à la distraction ou la compression rachidienne ou au transport osseux.

### Exposé de l'invention

A ces fins, l'invention se rapporte, selon un premier aspect, à un dispositif de déplacement de tissus à l'intérieur de l'organisme, notamment de tissus osseux, ce dispositif comprenant une première pièce, dite pièce de référence ; une seconde pièce, dite pièce de transport, montée coulissante par rapport à la pièce de référence; une tige comportant au moins un filetage, dite tige filetée, montée pivotante par rapport à la pièce de référence ; un arbre de commande ; des moyens d'entraînement reliant l'arbre de commande à la tige filetée ; un moyen de liaison, dit écrou de liaison, entre la pièce de transport et la tige filetée, cet écrou de liaison étant monté sur la tige filetée et étant guidé en rotation par rapport à la pièce de référence; des moyens de transformation du déplacement de l'écrou de liaison le long de la tige filetée en un déplacement de la pièce de transport par rapport à la pièce de référence, ce dispositif comprenant, pour limiter la translation longitudinale de ladite tige filetée par rapport à la pièce de référence, une première butée et une seconde butée solidaires de la tige filetée, ces butées coopérant respectivement avec un premier appui et un second appui solidaires de la pièce de référence , ces appuis étant placés à distance l'un de l'autre entre lesdites butées, l'écrou de liaison étant apte à se déplacer le long de la tige filetée entre le premier appui et le second appui.

La distance entre les deux butées est avantageusement supérieure à celle entre les appuis, de manière à permettre un jeu en translation de la tige filetée par rapport à la pièce de référence.

En fonctionnement normal, l'arbre de commande est entraîné en rotation par tout moyen connu par l'homme du métier. Par exemple, l'arbre de commande peut être associé à un aimant permanent qui, lorsque soumis à un champ magnétique, pivote pour s'orienter dans le champ et entraîne l'arbre de commande. En variante, l'arbre de commande est relié à un moteur, un moto-réducteur ou à un engrenage à clé.

Le dispositif présente, selon diverses réalisations, les caractères suivants, le cas échéant combinés.

Les moyens d'entraînement comportent un arbre de transmission flexible et/ou au moins un ressort hélicoïdal et/ou un joint de transmission, une chaîne de joints de transmission, un joint de cardans ou une chaîne de joints de cardans.

Les moyens d'entraînement comportent un dispositif à intermittence, notamment croix de Malte, solidaire de la tige filetée et entraîné en mouvement par l'arbre de commande, ce dispositif à intermittence étant apte à transformer un mouvement de rotation continue de l'arbre de commande en un mouvement intermittent de la tige filetée.

Les moyens de transformation comportent un cliquet ou une liaison rigide entre l'écrou de liaison et la pièce de transport ou un appui solidaire de l'écrou de liaison, cet appui coopérant avec une butée solidaire de la pièce de transport.

Le guidage en rotation de l'écrou de liaison par rapport à la pièce de référence peut être un guidage linéaire ou bien un guidage hélicoïdal tel que ledit écrou de liaison tourne d'un angle compris entre 10 et 180° quand il se déplace dudit premier audit second appui ou vice-versa.

Avantageusement, le dispositif comprend deux pièces de transport et deux tiges filetées. Avantageusement, les filetages des deux tiges filetées ont des caractéristiques de diamètre, de sens ou de pas différentes. Chaque tige filetée est avantageusement pourvue d'un dispositif à intermittence entrainé en mouvement par un arbre de commande, chaque dispositif à intermittence étant apte à transformer un mouvement de rotation continu de l'arbre de commande en un mouvement intermittent de chaque tige filetée, les deux dispositifs à intermittence étant montés en opposition, de sorte que lorsqu'un des dispositifs est en état d'entrainement, l'autre est en état de blocage.

Le diamètre de la (ou des) tige(s) filetée(s) est inférieur à quatre mm, notamment compris entre un et trois millimètres.

Lorsque le dispositif comprend deux pièces de transport, elles sont avantageusement entraînées par un arbre de commande commun. Les pièces de transport sont sensiblement parallèles entre elles ou non.

Avantageusement, les pièces de transport sont sensiblement de forme cylindrique et d'un diamètre usuel en chirurgie rachidienne, notamment compris entre trois et sept millimètres.

Dans certaines mises en oeuvre, les pièces de transport sont pourvues d'une partie sensiblement en forme de plaques comprenant des orifices de passage de vis usuelles en orthopédie des os longs ou en chirurgie maxillo-faciale.

Avantageusement, l'arbre de commande comprend un aimant permanent dont la direction de magnétisation est sensiblement perpendiculaire à l'axe de rotation de l'arbre de commande. L'arbre de commande comprend notamment un aimant permanent à base de terres rares, plus particulièrement Néodyme Fer Bore.

L'arbre de commande peut être relié à un moteur, un moto-réducteur ou un engrenage à clé. Avantageusement, l'arbre de commande comprend un cylindre creux dans lequel est fixé un aimant au moyen d'un adhésif à base de silicone.

Avantageusement le dispositif est au moins en partie réalisé par une technique de fabrication électrochimique, notamment la technique EFAB.

Avantageusement, la pièce de référence comprend un fourreau dans lequel coulisse un chariot de transport osseux sur lequel une pièce de transport est montée. Le chariot de transport osseux est pourvu d'un perçage longitudinal taraudé traversant, distant de l'axe du fourreau, ce perçage étant complémentaire du filetage de la tige filetée. Le chariot de transport osseux est pourvu d'un perçage diamétral logeant une vis à os formant pièce de transport, cette vis à os étant montée coulissante dans deux gorges parallèles longitudinales du fourreau.

Dans certaines mises en oeuvre, le dispositif comprend deux pièces de transport montées coulissantes en sens opposés l'une de l'autre par rapport à la pièce de référence. Avantageusement, le dispositif comprend une seule tige filetée, les moyens de transformation étant aptes à transmettre un déplacement de l'écrou de liaison dans un premier sens de déplacement, en un coulissement d'une première pièce de transport par rapport à la pièce de référence jusque vers une position extrême, le dispositif comprenant des moyens de verrouillage de la première pièce de transport dans cette position extrême. Les moyens de transformation sont aptes à transmettre un déplacement de l'écrou de liaison dans un deuxième sens de déplacement opposé audit premier sens, en un coulissement d'une deuxième pièce de transport par rapport à la pièce de référence. Avantageusement, les moyens de transformation et de verrouillage comprennent un système à cliquet.

L'invention trouve application en tant que clou médullaire ou plaque d'allongement ou de transport osseux notamment pour les os longs, les os plats et ceux de la mâchoire ou du crâne.

L'invention trouve également application en tant que tige de distraction ou de compression pour la correction du rachis ou du thorax, ou en tant que prothèse de croissance.

L'invention trouve en outre application dans la modification de tissus mous, notamment allongement d'une partie de l'intestin ou anneaux gastriques, ou bien encore dans la modification des sections des conduits du système sanguin, notamment cerclages artériels, anneaux de valvuloplastie.

### Description sommaire des dessins

D'autres objets et avantages de l'invention apparaîtront à la lumière de la description faite ci-après en référence aux dessins annexés dans lesquels :
- la figure 1 est une vue éclatée en perspective d'un dispositif de déplacement de tissus selon un premier mode de réalisation ;
- la figure 2 est une vue en perspective du dispositif assemblé de la figure 1 ;
- la figure 3 est une vue similaire à celle de la figure 2 dans laquelle l'intérieur du dispositif est visible ;
- la figure 4 est une vue en coupe du dispositif de la figure 2 dans une première position ;
- la figure 5 est une vue en coupe du dispositif de la figure 2 dans une deuxième position ;
- la figure 6 est une vue éclatée en perspective d'un dispositif de déplacement de tissus selon un deuxième mode de réalisation ;
- la figure 7 est une vue en perspective du dispositif assemblé de la figure 6 ;
- la figure 8 est une vue similaire à celle de la figure 7 dans laquelle l'intérieur du dispositif est visible ;
- la figure 9 est une vue en coupe du dispositif de la figure 6 dans une première position ;
- la figure 10 est une vue en coupe du dispositif de la figure 6 dans une deuxième position ;
- la figure 11 est une vue en perspective d'un dispositif de déplacement de tissus selon un troisième mode de réalisation ;
- la figure 12 est une vue en coupe du dispositif de la figure 11 dans une première position ;
- la figure 13 est une vue en coupe du dispositif de la figure 11 dans une deuxième position ;
- les figures 14 à 18 sont des vues de face d'un dispositif à intermittence en liaison avec un arbre de commande, dans cinq positions différentes ;
- la figure 19 est une vue en perspective d'un dispositif de déplacement de tissus selon un quatrième mode de réalisation ;
- la figure 20 est une vue similaire à la figure 19 dans laquelle l'intérieur du dispositif est visible ;
- la figure 21 est une vue éclatée en perspective du dispositif représenté en figures 19 et 20 ;
- les figures 22 à 26 sont des vues en coupe du dispositif de la figure 19, dans cinq positions différentes.

### Manières de réaliser l'invention

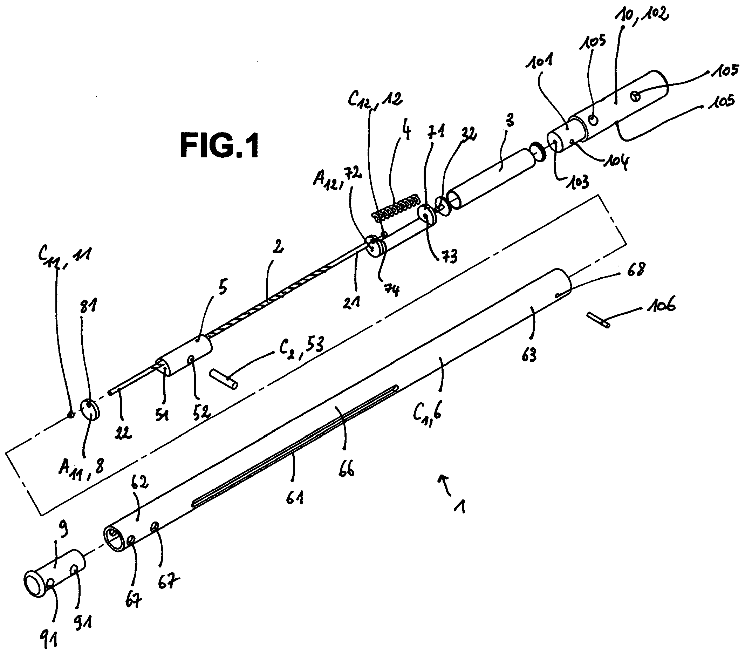

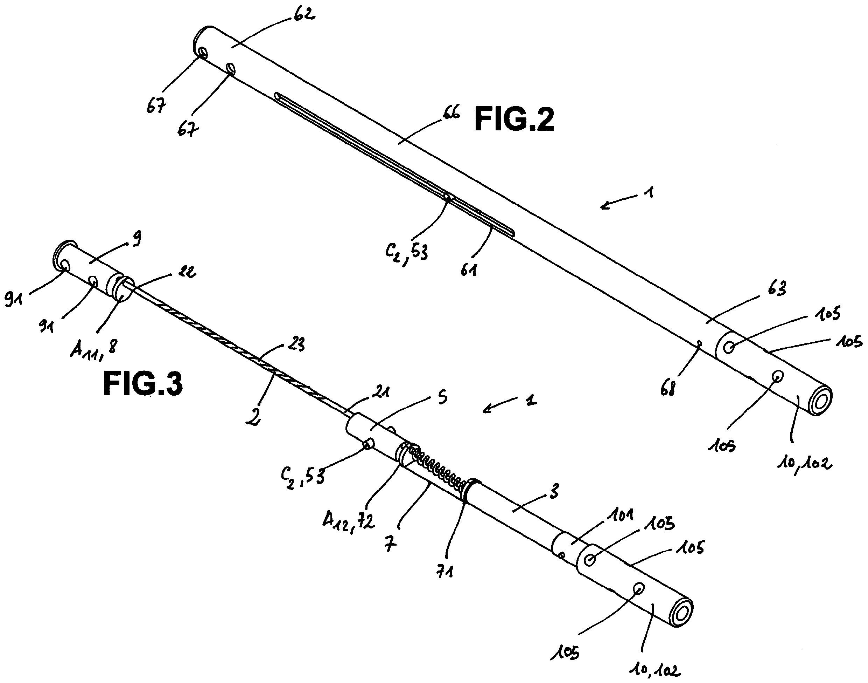

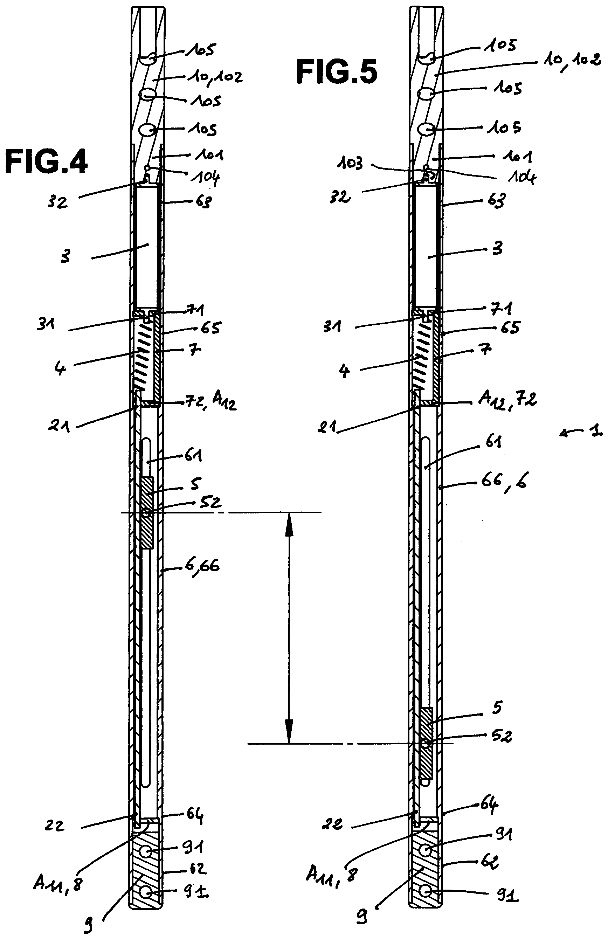

On se reporte maintenant aux figures 1 à 5 qui représentent un premier mode de réalisation, bien adapté au transport ou à l'allongement osseux.

Le clou 1 de transport osseux comprend une tige 2 filetée, un arbre 3 de commande, un arbre 4 flexible de transmission entre la tige 2 filetée et l'arbre 3 de commande. La première pièce C₁ dite pièce de référence, comprend un fourreau 6, dans lequel coulisse un chariot 5 de transport osseux.

La tige 2 filetée comporte deux parties 21, 22 extrêmes, de part et d'autre d'une portion 23 filetée, une partie 21 extrême étant lisse.

L'arbre 3 de commande se présente sous la forme d'un cylindre creux, dans lequel est fixé un aimant, par exemple au moyen d'une colle à base de silicone. A chacune de ses extrémités, l'arbre 3 de commande comprend un ergot axial 31, 32.

L'arbre 4 flexible de transmission est placé entre les bords d'une pièce 7 en forme d'étrier en U dont les joues 71, 72 en regard sont percées longitudinalement. Le perçage 73 d'une première joue 71 de l'étrier 7 loge un ergot axial 32 de l'arbre 3 de commande. Le perçage 74 de la deuxième joue 72 de l'étrier 7 est parallèle à et distant de l'axe du fourreau 6 et loge la partie 21 extrême lisse de la tige 2.

Dans une mise en oeuvre, l'arbre 4 flexible de transmission est du type de ceux commercialisés par la société SUHNER. L'arbre flexible de transmission se présente sous la forme d'un ou plusieurs ressorts hélicoïdaux enroulés autour d'un même axe central. Une gaine flexible peut entourer l'arbre flexible pour le protéger.

Sur les figures 1 à 5, un unique ressort est représenté. Toutefois, l'arbre 4 flexible de transmission peut être formé de plusieurs couches de ressorts hélicoïdaux enroulés les uns sur les autres, chaque couche pouvant comprendre plusieurs ressorts identiques juxtaposés. De préférence, les spires des ressorts d'une même couche ne sont pas jointives, afin de limiter les déformations et frottements engendrant des pertes, et de permettre un faible rayon de courbure de l'arbre flexible. Avantageusement, le pas des ressorts est juste supérieur ou égal au rayon intérieur le plus faible souhaité de la transmission flexible multiplié par π et divisé par le nombre de ressorts sur la couche considérée et le diamètre du fil constituant lesdits ressorts.

Le chariot 5 de transport se présente sous forme d'un cylindre tronqué par un plan longitudinal. Le chariot 5 est pourvu d'un perçage longitudinal 51 taraudé traversant, distant de l'axe du fourreau 6, ce perçage 51 étant complémentaire du filetage de la portion 23 filetée de la tige 2.

Le chariot 5 est pourvu d'un perçage diamétral 52 logeant une vis 53 à os.

Le fourreau 6 se présente sous la forme d'une pièce cylindrique creuse, munie de deux gorges 61 parallèles longitudinales, placées en vis-à-vis dans lesquelles coulisse la vis 53 à os.

Le chariot 5 forme moyen de liaison, dit écrou de liaison, entre la pièce de transport C₂ (vis à os 53) et la tige filetée 2, le chariot 5 étant monté sur la tige fileté 2 et guidé en rotation par rapport au fourreau 6 appartenant à la pièce de référence C₁.

Les moyens de transformation du déplacement de l'écrou de liaison (le chariot 5) en un déplacement de la pièce de transport (vis à os 53) sont formés par la liaison rigide entre l'écrou de liaison et la pièce de transport.

L'axe de rotation de la tige 2 est défini par le perçage 74 de la deuxième joue 72 de l'étrier 7 et par un perçage 81 d'une rondelle 8 d'appui.

Le fourreau 6, fermé à une extrémité 62 distale par une pièce 9 d'attache distale sous forme d'un bouchon et à une extrémité 63 proximale par une pièce 10 d'attache proximale, forme un logement pour l'ensemble des éléments mobiles du clou de transport osseux 1.

A partir de son extrémité 62 distale, le fourreau 6 présente une région 64 interne distale, de section complémentaire de celle de la rondelle 8 d'appui.

A partir de son extrémité 63 proximale, le fourreau 6 présente une région 65 interne proximale, de section complémentaire de celle des joues 71, 72 de l'étrier 7.

Dans une région 66 interne intermédiaire, l'intérieur du fourreau 6 présente une section sensiblement circulaire, de diamètre inférieur à celui de la rondelle 8 d'appui et des joues 71, 72 de l'étrier 7, de sorte que l'intérieur du fourreau 6 forme deux butées, respectivement entre la région 64 distale et la région 66 intermédiaire et entre la région 66 intermédiaire et la région 65 proximale.

Sur sa partie 62 extrême distale, le fourreau 6 comprend deux perçages 67 diamétraux distaux, et sur sa partie 63 extrême proximale, le fourreau 6 comprend un perçage 68 diamétral proximal.

La pièce 9 d'attache distale, se présente sous la forme d'un bouchon cylindrique, de diamètre sensiblement égal à celui de la section de la région 64 interne distale du fourreau 6. Cette pièce 9 d'attache distale est munie de deux perçages 91 diamétraux.

La pièce 10 d'attache proximale comprend deux portions 101, 102. La première portion 101 de la pièce 10 d'attache proximale est cylindrique, de diamètre sensiblement égal à celui de la section de la région 65 interne proximale du fourreau 6, et comprend un perçage 103 longitudinal et un perçage 104 diamétral.

La deuxième portion 102 de la pièce 10 d'attache proximale est cylindrique, de diamètre supérieur à celui de la première portion 101, et est munie de trois perçages 105 diamétraux, répartis longitudinalement et décalés angulairement de 90° et aptes à recevoir des vis à os.

Le montage du clou 1 peut être obtenu comme suit.

La tige 2 filetée est insérée dans le perçage longitudinal 51 taraudé du chariot 5 de transport osseux. La partie extrême 21 lisse de la tige 2 est également insérée dans le perçage 74 de la joue 72 de l'étrier 7. Ainsi, l'axe de rotation de la tige 2 filetée se trouve à distance de l'axe de symétrie du chariot 5 de transport osseux.

Un deuxième écrou 12 formant une deuxième butée C₁₂ axiale est vissé et bloqué en rotation, par exemple par soudure laser, sur la partie extrême 21 de la tige 2 et est placé contre la deuxième joue 72.

La joue 72 forme un deuxième appui A₁₂, solidaire de la pièce de référence C₁ de sorte que la tige 2 est bloquée en translation longitudinale suivant une première direction, tout en restant libre en rotation.

Un ergot 32 à une extrémité de l'arbre 3 de commande est monté pivotant dans le perçage 73 de la première joue 71 de l'étrier 7.

L'arbre 4 flexible est alors monté entre les deux joues 71, 72 de l'étrier 7, les extrémités de l'arbre 4 étant soudées respectivement à la partie 21 extrême lisse de la tige 2 filetée et à l'ergot 32 de l'arbre 3 de commande.

La rondelle 8 d'appui est alors insérée dans l'extrémité 62 distale du fourreau 6, jusqu'à venir en butée à la limite de la région 66 interne intermédiaire.

L'ensemble constitué, à savoir la tige 2 filetée, le chariot 5 de transport, l'étrier 7, l'arbre 4 de transmission et l'arbre 3 de commande, est alors inséré dans le fourreau 6, la deuxième extrémité 22 de la tige 2 filetée étant insérée dans le perçage 81 de la rondelle 8 d'appui. L'étrier 7 est placé jusqu'au fond de la région 65 interne proximale du fourreau 6, tandis que la portion 23 filetée de la tige 2 et le chariot 5 sont placés dans la région 66 interne intermédiaire du fourreau 6.

Un premier écrou 11 formant une première butée C₁₁ axiale est vissé et soudé, par exemple par laser, sur la deuxième extrémité 22 de la tige 2 filetée, contre la rondelle 8.

La rondelle 8 forme un premier appui A₁₁, solidaire de la pièce de référence C₁, et la tige 2 est ainsi bloquée en translation longitudinale suivant une seconde direction, tout en restant libre en rotation.

La disposition des appuis A₁₁, 8 et A₁₂, 72, placés entre les butées C₁₁, 11 et C₁₂, 12, permet que la tige filetée 2 ne puisse être sollicitée qu'en traction.

Puis, le fourreau 6 est refermé à ses deux extrémités par les pièces 9, 10 d'attache.

A cette fin, le bouchon 9 est inséré dans la région 64 interne distale du fourreau 6, de sorte que ses deux perçages 91 diamétraux soient alignés avec les perçages 67 distaux du fourreau 6 et puissent recevoir des vis à os. La première portion 101 cylindrique de la pièce 10 d'attache proximale est insérée dans la région 65 interne proximale du fourreau 6, le deuxième ergot 31 de l'arbre 3 de commande s'insérant dans le perçage 103 longitudinal de la pièce 10 d'attache proximale. Puis, le perçage 68 proximal du fourreau 6 est aligné avec le perçage 104 diamétral de la première portion 101 cylindrique. Un ergot 106 assure le blocage de la pièce 10 d'attache proximale par rapport au fourreau 6. Des soudures laser renforcent ces assemblages.

Ainsi, le fourreau 6 et les pièces 9, 10 d'attache constituent une première pièce C₁, dite pièce de référence, la vis à os 53 formant une deuxième pièce C₂, dite de transport, les butées C₁₁ et C₁₂ de la tige 2 filetée correspondent aux écrous 11, 12 de butée axiale placés contre la rondelle A₁₁, 8 d'appui et contre la deuxième joue A₁₂, 72 de l'étrier 7. L'écrou de liaison est formé par le chariot 5 et la transmission du déplacement de ce chariot 5 à pièce de transport est obtenue par la liaison rigide entre l'écrou de liaison et la pièce de transport.

Un exemple de fonctionnement du clou osseux 1 est donné ci-après.

Un os, par exemple suite à l'ablation d'une tumeur, comprend une portion manquante, entre deux portions extrémales saines. Une première portion saine subit une ostéotomie, de manière à libérer une rondelle entre les deux portions extrémales saines. Les deux portions et la rondelle sont alésées, de façon à permettre le passage du clou 1.

Le clou 1 de transport osseux est dans une position initiale, par exemple telle que le chariot 5 de transport est plus proche de l'étrier 7 que de la rondelle A₁₁, 8 d'appui, l'étirement du cal osseux se faisant de l'étrier 7 vers la rondelle A₁₁, 8 d'appui. Par ailleurs, le chariot 5 est avantageusement positionné de sorte que son perçage 52 diamétral soit en vis-à-vis des gorges 61 longitudinales du fourreau 6.

La pièce 10 d'attache proximale est fixée dans la première portion extrémale d'os, au moyen de trois vis à os passées dans les perçages 105 de la deuxième portion 102 cylindrique de la pièce 10.

La partie distale du clou est fixée dans la deuxième portion extrémale d'os, au moyen de deux vis à os passées dans les perçages 67 distaux du fourreau 6 et dans les perçages 91 de la pièce d'attache distale 9.

Une vis 53 à os permet d'attacher la rondelle d'os au chariot 5 au travers du perçage 52 diamétral du chariot 5 et des gorges 61 du fourreau 6.

L'arbre 3 de commande est ensuite pivoté d'une valeur connue. Sa rotation est transmise à la tige 2 filetée via l'arbre 4 flexible, provoquant le déplacement du chariot 5 vers la rondelle A₁₁, 8 d'appui, la vis C₂, 53 à os fixée à la rondelle d'os glissant le long des gorges 61 du fourreau 6 et assurant le guidage en translation du chariot 5 de transport, de sorte que la rondelle d'os est éloignée progressivement de la première portion extrémale d'os.

En effectuant cette opération régulièrement, on étire le cal osseux se formant entre la rondelle d'os et la première portion extrémale d'os, jusqu'à compléter la portion manquante d'os.

Une fois la jonction entre la rondelle d'os et la seconde portion extrémale d'os et le cal osseux devenus solides, le dispositif 1 peut être laissé dans l'os ou retiré par une opération chirurgicale.

L'arbre 4 flexible de transmission permet avantageusement de désaxer le taraudage 51 du chariot 5 de transport par rapport au reste du clou osseux 1, de sorte que la vis C₂, 53 à os fixée à la rondelle d'os peut être insérée suivant un diamètre du clou 1.

L'arbre 4 flexible de transmission permet, en outre, de former un angle non nul entre l'arbre 3 de commande et la tige 2 filetée, afin d'adapter la forme du clou 1 de transport osseux à son utilisation, par exemple dans le cas d'une fixation sur la mâchoire pour un transport ou un allongement.

Un allongement peut être obtenu en ne fixant pas la partie distale du clou, qui ne présente dans ce cas pas de trou pour ce faire.

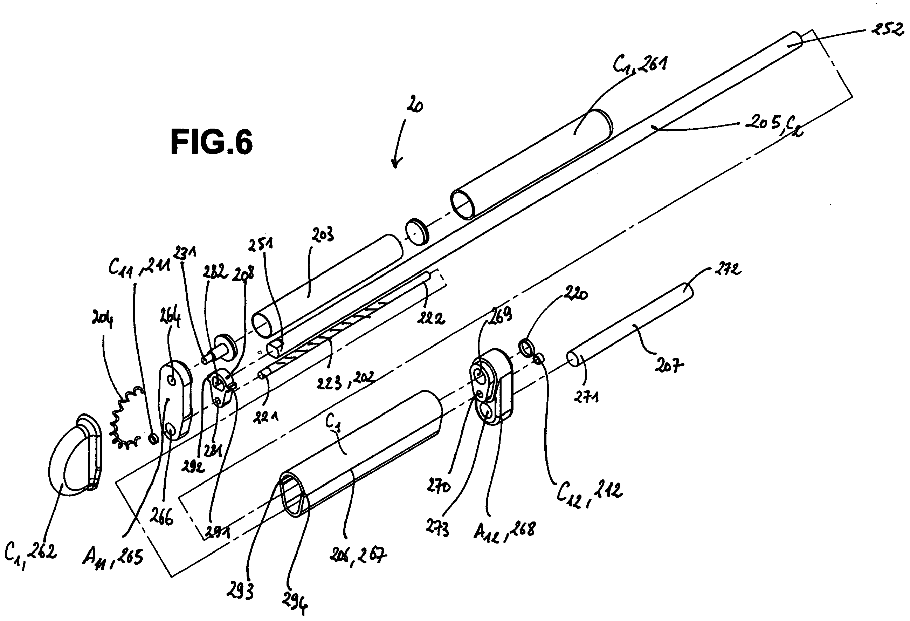

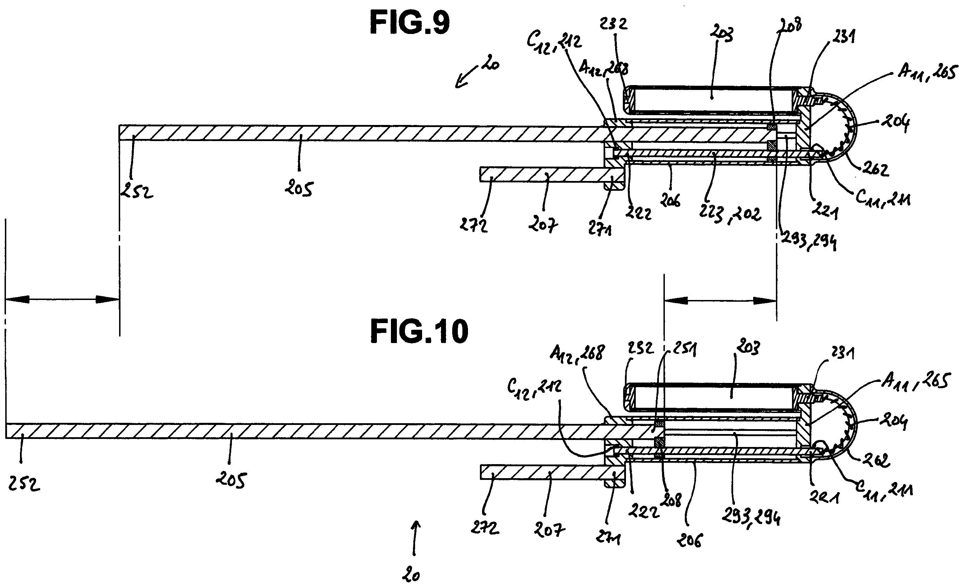

Un deuxième mode de réalisation est représenté sur les figures 6 à 10.

Ce mode de réalisation est plus particulièrement adapté à la réalisation de tiges de distraction ou de compression, qui peuvent être liées à des vertèbres, des côtes ou au bassin, pour corriger le rachis ou le thorax. C'est la raison pour laquelle, dans la suite de cette description, il sera essentiellement fait référence à cette application.

La tige 20 de distraction comprend une tige 202 filetée, un arbre 203 de commande, un arbre 204 flexible de transmission entre la tige 202 filetée et l'arbre 203 de commande, une tige 205 mobile et un boîtier 206 logeant la tige filetée 202 et les arbres 203, 204.

Le boîtier 206 forme première pièce C₁, dite pièce de référence. Une tige 207 fixe est montée sur le boîtier 206. La tige 205 mobile forme deuxième pièce C₂, dite pièce de transport.

La tige filetée 202 et l'arbre 203 de commande ne sont pas coaxiaux. Dans le mode de réalisation représenté, l'axe de rotation de la tige filetée 202 est sensiblement parallèle à l'axe de rotation de l'arbre de commande 203. La tige 205 mobile est montée coulissante, à l'intérieur du boîtier 206, l'axe de coulissement de la tige 205 mobile étant sensiblement disposé dans le plan contenant les axes de rotation de la tige filetée 202 et de l'arbre de commande 203.

Dans le mode de réalisation représenté, l'axe de coulissement de la tige 205 mobile s'étend entre les axes de rotation de la tige filetée 202 et de l'arbre de commande 203.

L'arbre 203 de commande se présente sous la forme d'un cylindre creux, dans lequel est fixé un aimant, par exemple au moyen d'une colle à base de silicone. L'arbre 203 de commande comprend à chacune de ses extrémités, dans l'alignement, un ergot 231, 232 placé dans l'axe de l'arbre 203.

La tige 205 mobile comprend une partie 251 extrême proximale et une partie 252 extrême distale. Les termes « distal », « proximal » sont employés ici en référence au boîtier C₁, 206.

La partie 252 extrême distale de la tige 205 mobile est apte à recevoir des moyens de liaison (non représentés), tels que des crochets, des vis ou des rubans, aux os (en particulier vertèbres, côtes ou bassin) auxquels on souhaite la fixer.

La tige 207 fixe comprend une partie 271 extrême proximale et une partie 272 extrême distale. La partie 271 extrême proximale de la tige 207 fixe est encastrée dans le boîtier C₁, 206. La partie 272 extrême distale de la tige 207 fixe est apte à recevoir des moyens de liaison (non représentés), tels que des crochets, des vis ou des rubans, aux os (en particulier vertèbres, côtes ou bassin) auxquels on souhaite la fixer.

Dans le mode de réalisation représenté, la tige 205 mobile et la tige 207 fixe sont sensiblement droites et parallèles et peuvent être recoupées et courbées par le chirurgien. Dans d'autres modes de réalisation, non représentés, au moins l'un de ces deux éléments 205, 207 est fourni cintré.

La tige 202 filetée est pourvue de deux parties 221, 222 extrêmes lisses, de part et d'autre d'une portion 223 filetée.

Un palet 208 forme moyen de liaison entre la pièce de transport C₂, 205 et la tige filetée 202. Ce palet 208, ou écrou de liaison, comprend un taraudage 281 complémentaire de la portion 223 filetée de la tige 202. L'extrémité 251 proximale de la tige 205 mobile est conformée pour s'insérer, par exemple à force, dans une ouverture 282 complémentaire du palet 208. Des protubérances 291,292 solidaires dudit palet 208 et sensiblement symétriques par rapport à l'ouverture 282, coopèrent respectivement avec des gorges 293,294 pratiquées à l'intérieur du boîtier C₁, 206 pour assurer le guidage en rotation de l'écrou de liaison 208 par rapport à la pièce de référence C₁.

Quand, en plus d'une distraction ou d'une compression, on souhaite produire une torsion, lesdites protubérances 291,292 et lesdites gorges 293,294 peuvent avantageusement être hélicoïdales, forçant la rotation progressive de la tige 205 mobile à mesure qu'elle se déplace par rapport au boîtier C₁, 206 et, ainsi, la torsion de la portion de colonne vertébrale traitée. De manière similaire, un guidage hélicoïdal de l'écrou de liaison est intéressant quand, en plus d'allonger un os, on souhaite simultanément corriger une déformation en torsion de cet os.

L'arbre 204 de transmission flexible relie un premier ergot 231 de l'arbre 203 de commande à l'extrémité lisse 221 de la tige 202 filetée.

Le boîtier C₁, 206 comprend un fourreau 261 cylindrique dans lequel se loge l'arbre 203 de commande, le deuxième ergot 232 de l'arbre 303 de commande s'insérant dans une réservation du fourreau 261.

Le premier ergot 231 de l'arbre 203 de commande passe par une première ouverture 264 d'une pièce 265 de support, fixée au fourreau 261 cylindrique par exemple par emboîtement et soudure.

Une première extrémité 221 de la tige 202 filetée est insérée dans une deuxième ouverture 266 de la pièce 265 de support, de façon à ce quelle émerge d'un même côté que le premier ergot 231 de l'arbre 203 de commande. La portion 223 filetée de la tige 202 s'étend alors au moins partiellement du côté opposé de la pièce 265 de support.

Un premier écrou 211 forme une première butée C₁₁ axiale de la tige filetée 202. Cet écrou 211, C₁₁ est vissé et soudé, par exemple par soudage laser, sur la première extrémité 221 de la tige 202 filetée contre la pièce 265 de support.

La pièce 265 de support forme un premier appui A₁₁, solidaire de la pièce de référence C₁, 206.

L'arbre 204 flexible est fixé, par exemple par soudage, sur le premier ergot 231 de l'arbre 203 de commande et sur la première extrémité 221 de la tige 202 filetée. L'arbre 204 flexible décrit alors une courbe, sensiblement en arc de cercle ou en U.

Le boîtier C₁, 206 comprend en outre une coque 262, formant un logement pour l'arbre 204 de transmission flexible. La coque 262 est fixée à son tour à la pièce A₁₁, 265 de support.

Le palet 208 s'insère dans le fourreau 267, de préférence avec un léger jeu. Le palet 208 coopère avec la tige 202 filetée, via le taraudage 281, est fixé sur la tige C₂, 205 mobile et est guidé en rotation par l'intermédiaire des protubérances 291,292 coopérant avec les gorges 293,294 du fourreau 267, partie du boîtier C₁.

Le fourreau 267 est assemblé à l'une de ses extrémités contre la pièce A₁₁, 265 de support. L'autre extrémité du fourreau 267 est fermée par un capot 268, comprenant une première ouverture 269 laissant le passage à la tige 205 mobile.

Le capot 268 comprend, en outre, une deuxième ouverture 270 accueillant la deuxième partie 222 extrême de la tige 202 filetée.

Le capot 268 forme un deuxième appui A₁₂, solidaire de la pièce de référence 206, C₁.

Un écrou 212 de blocage axial forme deuxième butée C₁₂ est inséré sur la deuxième extrémité 222 de la tige 202 filetée contre le capot 268, A₁₂.

La partie 271 extrême proximale de la tige 207 fixe est rendue solidaire du capot A₁₂, 268, par exemple en l'insérant en forçant dans une troisième ouverture 273 du capot A₁₂, 268 et en soudant en bout.

Les deux fourreaux 261, 267, la coque 262, la pièce 265 de support et le capot A₁₂, 268 formant le boîtier C₁, 206 sont assemblés par exemple par soudures laser ou collage.

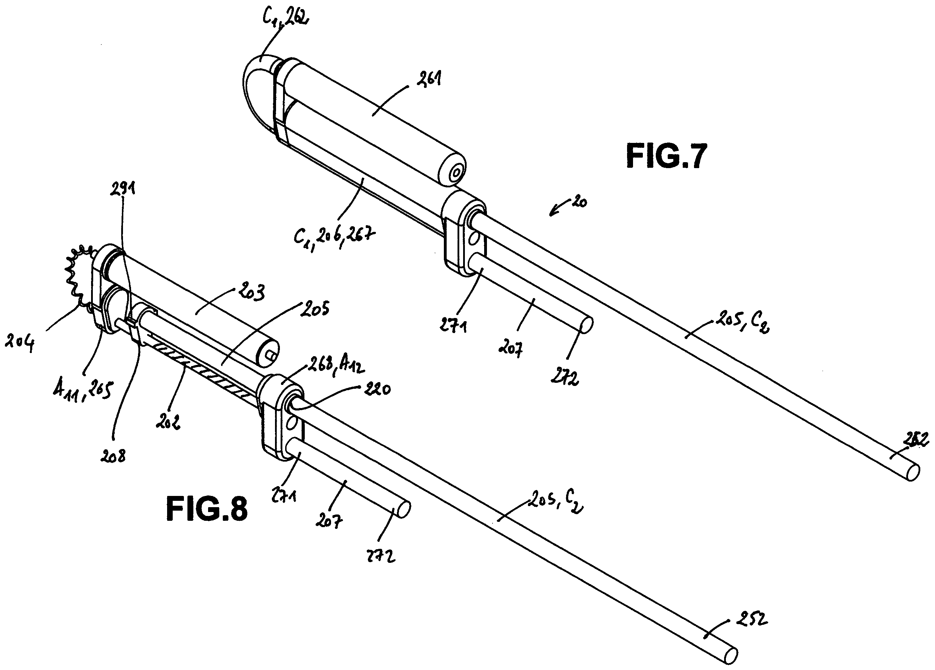

Ainsi qu'il apparaît sur les figures 7 à 10 notamment, la tige 20 de distraction présente une forme générale en J, le boîtier C₁, 206 formant la partie rigide et non conformable du dispositif pouvant être avantageusement placé en dessus ou en dessous de la zone concernée par le traitement chirurgical.

La tige 207 fixe peut aussi être fixée au capot 268 dans la direction opposée pour former une tige 20 en forme de I, mieux adaptée si elle doit être fixée très haut et très bas sur le rachis.

Le boîtier 206 forme une première pièce C₁, dite pièce de référence, la tige 205 mobile forme une pièce C₂ de transport, les butées C₁₁ et C₁₂ de la tige 202 filetée sont formées par les écrous 211, 212 de butée axiale placés contre les appuis A₁₁ et A₁₂ formés respectivement par la pièce 265 de support et le capot 268 du boîtier C₁, 206.

Le palet 208, ou écrou de liaison, forme moyen de liaison entre la pièce de transport C₂, 205 et la tige filetée 202. Les moyens de transformation du déplacement de l'écrou de liaison 208 en un déplacement de la pièce de transport C₂, 205 sont formés par une liaison rigide entre l'écrou de liaison 208 et la pièce de transport C₂, 205, par exemple par insertion à force de la partie extrême 251 de la pièce de transport C₂, 205 dans l'ouverture 282 du palet 208.

Un exemple de fonctionnement du distracteur 20 est donné ci-après.

La partie 252 extrême distale de la tige 205 mobile est fixée par exemple à une première vertèbre, et la partie 272 extrême distale de la tige 207 fixe est fixée à une deuxième vertèbre, par exemple située sous la première vertèbre. Les fixations se font du coté concave ou convexe d'une courbure, selon le cas et selon que l'on souhaite appliquer à la portion de rachis traitée une distraction ou une compression respectivement.

Un champ magnétique dans lequel l'aimant s'oriente est appliqué : l'aimant pivotant entraîne l'arbre 203 de commande. Le pivotement de l'arbre 203 de commande est transmis à la tige 202 filetée via l'arbre 204 de transmission flexible. Le palet 208, en liaison hélicoïdale avec la tige 202 filetée, se déplace le long de la portion 223 filetée de la tige 202, entraînant la tige C₂, 205 mobile dans le sens d'une distraction ou d'une compression.

Une bague de visualisation 220 est avantageusement soudée à la tige C₂, 205 mobile et permet de visualiser facilement le mouvement accompli, sur une radiographie.

Les deux vertèbres sont ainsi progressivement éloignées ou rapprochées, induisant une modification de la courbure du rachis.

Avantageusement, la capacité à évoluer dans les deux sens de la tige 20 est utilisée pour périodiquement relâcher l'effort appliqué sur le rachis, de manière à lui rendre par moments provisoirement une partie de sa mobilité et ainsi retarder la dégénérescence des disques et la fusion spontanée, généralement observée après quelques années de maintien avec les matériels fixes connus.

Dans le cas où la variation de longueur de la tige 20 s'accompagne d'une rotation, la partie 252 extrême distale de la tige 205 mobile est fixée à une vertèbre, et la partie 272 extrême distale de la tige 207 fixe est fixée au bassin, s'il y a lieu de corriger une torsion du rachis par rapport au bassin.

Dans le cas, plus fréquent, du traitement d'une scoliose, on combine avantageusement sur le rachis à traiter deux tiges têtes bêches, de part et d'autre de l'apex de la courbe dont la partie 252 extrême distale de la tige 205 mobile est liée à cet apex, et la partie 272 extrême distale de la tige 207 fixe est fixée respectivement, pour l'une, à une vertèbre non ou peu tournée située au-dessus de l'apex, et, pour l'autre, à une vertèbre non ou peu tournée et située au-dessous de l'apex. De cette manière, on aide simultanément au redressement et à la détorsion de la scoliose.

Dans le mode de réalisation présenté, l'arbre 203 de commande est relié à une seule tige 205 mobile.

Dans une variante de réalisation, non représentée, le deuxième ergot 232 de l'arbre 203 de commande est également fixé à un deuxième arbre flexible de transmission, transmettant la rotation de l'arbre de commande à une deuxième tige filetée, provoquant le déplacement d'une deuxième tige mobile C₂. Dans une mise en oeuvre, cette deuxième tige mobile coulisse dans la direction de déplacement de la première tige mobile C₂, 205 et dans le sens opposé. Dans une autre mise en oeuvre, cette deuxième tige mobile coulisse dans une direction formant un angle avec la direction de déplacement de la première mobile C₂, 205.

L'arbre 204 de transmission flexible permet ainsi la réalisation facile et économique de tiges en forme de J ou de I, pouvant être indifféremment allongées ou raccourcies à tout moment et dont la partie droite non recoupable et recourbable par le chirurgien est de longueur compatible avec le placement sur le rachis ou le thorax.

Suivant un autre mode préféré de réalisation, les moyens d'entrainement reliant l'arbre de commande à la tige filetée comprennent un dispositif d'indexation ou dispositif à intermittence.

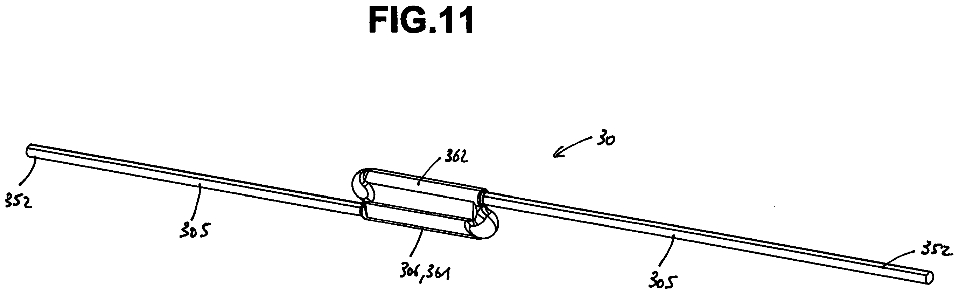

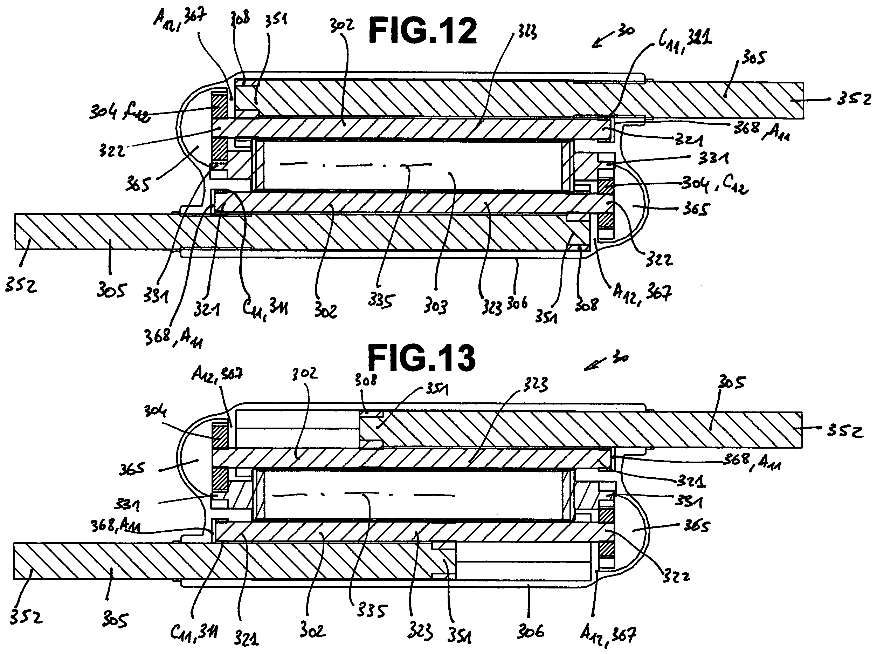

Ce mode préféré de réalisation va être présenté en faisant référence aux figures 11 à 18, dans lesquelles il est représenté une tige de distraction ou de compression comprenant deux tiges mobiles.

La tige 30 de distraction comprend deux tiges 302 filetées, un arbre 303 de commande, un dispositif 304 à intermittence entre chaque tige 302 filetée et l'arbre unique 303 de commande, deux tiges 305 mobiles et un boîtier 306.

Les tiges filetées 302 et l'arbre 303 de commande ne sont pas coaxiaux. Dans le mode de réalisation représenté, les axes de rotation des tiges filetées 302 sont sensiblement parallèles à l'axe de rotation de l'arbre de commande 303. Les tiges 305 mobiles sont montées coulissantes, à l'intérieur du boîtier 306, les axes de coulissement des tiges 305 mobiles étant sensiblement disposés dans le plan contenant les axes de rotation des tiges filetées 302 et de l'arbre de commande 303.

Dans le mode de réalisation représenté, l'axe de rotation de l'arbre de commande 303 est disposé à égale distance des axes de rotation des tiges filetées 302.

L'arbre 303 de commande se présente sous la forme d'un cylindre creux, dans lequel est fixé un aimant, par exemple au moyen d'une colle à base de silicone. L'arbre 303 de commande comprend à chacune de ses extrémités deux ergots 331, 332.

Un premier ergot 331 présente une section en croissant, placé sensiblement dans l'axe d'élancement de l'arbre 303 de commande. Le premier ergot 331 comprend une surface 333 périphérique concave et une surface 334 périphérique convexe. La surface 333 concave croise l'axe 335 d'élancement de l'arbre 303 de commande, tandis que la surface 334 convexe est coaxiale à l'axe 335 d'élancement.

Le deuxième ergot 332 s'étend sensiblement parallèlement à l'axe 335 d'élancement de l'arbre 303 de commande et à distance de cet axe 335.

Chaque tige 305 mobile comprend une partie 351 extrême proximale et une partie 352 extrême distale. Les termes « proximal » et « distal » sont employés ici en référence au boîtier 306.

La partie 352 extrême distale est apte à recevoir des moyens de liaison (non représentés), tels que des crochets, des vis ou des rubans, aux os (en particulier vertèbres, côtes ou bassin) auxquels on souhaite la fixer.

Dans le mode de réalisation représenté, les tiges 305 mobiles sont sensiblement droites et parallèles et peuvent être recoupées et courbées par le chirurgien. Dans d'autres modes de réalisation, non représentés, au moins l'une de ces tiges 305 mobiles peut être fournie cintrée.

Chaque tige 302 filetée est pourvue de deux parties 321, 322 extrêmes lisses, de part et d'autre d'une portion 323 filetée.

Ainsi qu'il apparaît sur les figures 11, 12 et 13, les deux tiges 305 mobiles s'étendent sensiblement symétriquement de part et d'autre de l'arbre de commande 303.

Chaque tige 302 filetée coopère avec un palet 308, muni d'un taraudage complémentaire de la portion 323 filetée de la tige 302. L'extrémité 351 proximale de chaque tige 305 mobile est conformée pour s'insérer, par exemple à force, dans une ouverture complémentaire d'un palet 308. Avantageusement, les palets 308 ne présentent aucune symétrie de révolution.

Le boîtier C₁, 306 illustré présente une symétrie centrale et comprend deux coques 361, 362 assemblées, par exemple par vissage. Le boitier 306 comprend deux ouvertures de passage pour les tiges 305 mobiles.

L'arbre 303 de commande est placé dans un logement central du boitier 306, les ergots 331, 332 de chaque extrémité débouchant dans un logement 365 extrême.

Le montage d'une tige 302 filetée et d'une tige 305 mobile avec un palet 308 dans le boîtier C₁, 306 se fait de la manière suivante.

Une première extrémité 321 de la tige 302 filetée est insérée dans une ouverture d'une paroi 367 dans le boîtier 306, de sorte à émerger dans un logement 365 extrême. La portion 323 filetée de la tige 302 s'étend alors au moins partiellement du côté opposé de la paroi 367. Une butée longitudinale, décrite plus loin, est réalisée entre la première partie 321 extrême de la tige et la paroi 367.

La deuxième partie 322 extrême de la tige 302 filetée est placée dans une réservation 368 du boîtier 306.

Un premier écrou 311 forme une première butée C₁₁ dans la réservation 368 formant appui A₁₁ pour bloquer longitudinalement la tige 302 filetée.

Le palet 308 coopérant avec la tige 302 filetée, via le taraudage 381, et solidaire de la tige 305 mobile s'insère en liaison glissière dans un logement de forme sensiblement complémentaire, et de préférence laissant un léger jeu. Comme dans la forme de réalisation précédente de l'invention, une liaison hélicoïdale peut être utilisée à la place de la liaison glissière, pour agir simultanément sur la courbure et la torsion d'une scoliose en liant chacune des tiges 305 mobiles de part et d'autre de l'apex de la courbure à des vertèbres pas ou peu tournées et le boîtier C₁ à cet apex.

Lorsque la rotation de l'arbre de commande 303 est transmise aux tiges 302 filetées, chaque palet 308 se déplace le long d'une tige 302 filetée, entraînant la tige 305 mobile solidaire du palet 308.

Dans chaque logement 365 extrême du boîtier 306, un dispositif 304 à intermittence est placé, pour transmettre la rotation de l'arbre 303 de commande à chaque tige 305.

Chaque dispositif 304 à intermittence comprend des moyens pour que, lorsque l'arbre 303 de commande effectue une rotation complète, la tige 302 filetée avec laquelle il coopère n'ait pivotée que dans un sens (horaire ou antihoraire), la tige 305 mobile correspondante ne s'étant déplacée que dans une seule direction.

Chaque dispositif 304 à intermittence peut prendre deux états.

Dans un premier état, dit d'entrainement, le dispositif 304 à intermittence transmet la rotation de l'arbre 303 de commande à la tige 302 filetée, pour provoquer le déplacement de la tige 305 mobile.

Dans un second état, dit de blocage, le dispositif à intermittence ne transmet pas la rotation de l'arbre 303 de commande à la tige 302 filetée, la tige 305 mobile restant immobile dans le boîtier 306.

Avantageusement, chaque dispositif 304 à intermittence comprend une croix de Malte 310, coaxiale avec la tige 302 filetée sur laquelle elle est montée solidaire, par exemple en insérant par forçage la première portion 321 extrême de la tige 302 filetée émergente dans le logement 365 extrême dans une ouverture centrale de la croix de Malte 310.

La croix 310 de Malte forme en outre une butée C₁₂ axiale contre la paroi 367 formant appui A₁₂, de sorte que chaque tige 302 filetée se retrouve bloquée en translation longitudinale par l'écrou C₁₁, 311 et la croix C₁₂, 310 de Malte en appui respectivement contre la réservation A₁₁, 368 et la paroi A₁₂, 367.

La croix 310 de Malte se présente sous la forme d'une roue dentée, dans laquelle les dents sont formées par une alternance de deux types d'encoches :
- une encoche 313 courte, en forme de demi-cercle, de rayon sensiblement égal à celui de la surface 334 périphérique convexe de l'ergot 331 en forme de croissant de l'arbre 303 de commande ;
- une encoche 314 longue, en forme de U, s'étendant radialement sur une plus grande profondeur que l'encoche 313 courte, et de largeur sensiblement égale au diamètre de l'ergot 332 désaxé de l'arbre 303 de commande.

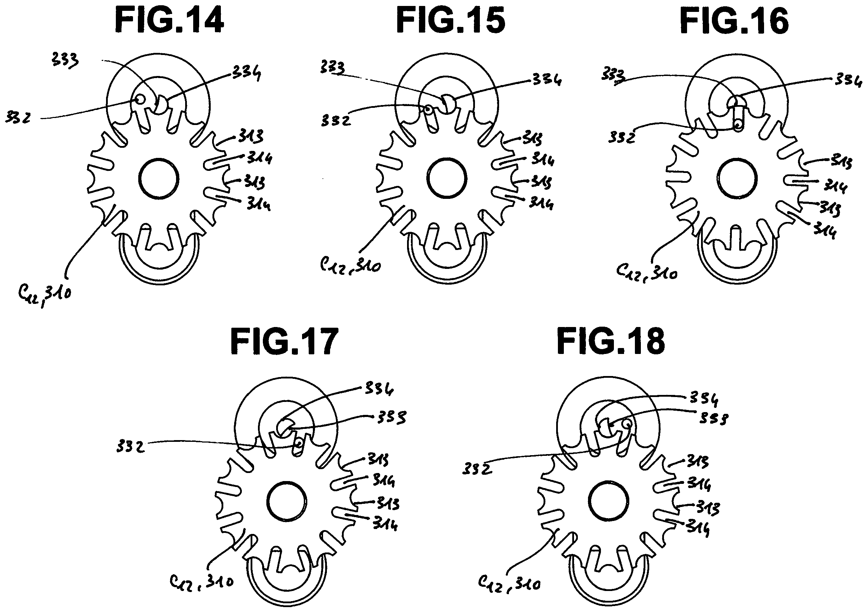

Le fonctionnement d'une croix 310 de Malte en coopération avec l'arbre 303 de commande est détaillé ici, en référence aux figures 14 à 18.

Dans une position de départ, un ergot 331 en forme de croissant sur une première extrémité de l'arbre 303 de commande est positionné dans une première encoche 313 courte de la croix, la surface 334 convexe étant en contact avec le fond de l'encoche 313. L'arbre 303 de commande pivote dans l'encoche 313 courte, sans entraîner la croix 310 : le dispositif 304 à intermittence est dans la position de blocage.

L'arbre 303 de commande continuant à pivoter, l'ergot 332 désaxé sur la première extrémité de l'arbre 303 de commande s'engage dans l'encoche 314 longue adjacente à la première encoche 313 courte, tandis que la surface 334 périphérique convexe de l'ergot 331 en forme de croissant quitte l'encoche 313 courte et que la surface 333 concave vient en vis-à-vis du fond de l'encoche 313 courte, libérant l'encoche 313 courte. L'ergot 332 désaxé entraine alors la croix 310 en rotation : le dispositif 304 à intermittence est dans la position d'entrainement.

L'arbre 303 de commande continue à pivoter, entraînant la croix 310, l'ergot 332 désaxé glissant le long de l'encoche 314 longue.

Lorsque l'ergot 332 désaxé quitte l'encoche 314 longue, la surface 334 périphérique convexe de l'ergot 331 en forme de croissant s'engage dans l'encoche 313 courte suivante, bloquant de nouveau la transmission.

Dans le mode de réalisation représenté, les deux tiges 305 mobiles se déplacent suivant deux directions parallèles et dans deux sens opposés. Toutefois, les tiges 305 mobiles peuvent se déplacer dans un même sens. Par ailleurs, il est possible de former un angle entre les tiges 305 mobile de chaque module d'élongation, par exemple en interposant un arbre de transmission flexible entre le dispositif 304 à intermittence et la première partie 321 extrême de la tige 302 filetée.

Les deux tiges 305 mobiles peuvent être de longueur et de section identiques ou non.

Les deux dispositifs à intermittence sont avantageusement montés en opposition. En d'autres termes, lorsqu'un des dispositifs 304 à intermittence est en état de transmission, l'autre dispositif 304 à intermittence est en état de blocage.

En particulier, dans le cas où les dispositifs 304 à intermittence comprennent chacun une croix 310 de Malte, celles-ci sont montées de sorte que lorsque l'ergot 331 en forme de croissant d'une extrémité de l'arbre 303 de commande est positionné dans une encoche 313 courte d'une première croix 310 de Malte, l'ergot 332 désaxé de l'autre extrémité est dans une encoche 314 longue de la deuxième croix 310 de Malte, et inversement.

Le boîtier 306 forme une première pièce C₁, dite pièce de référence. Chaque tige 305 mobile forme une deuxième pièce C₂, dite pièce de transport.

Les moyens de liaison, dits écrous de liaison, entre les pièces de transport C₂, 305 et les tiges filetées 303, sont formés par les palets 308, montés sur les tiges filetés 302 et guidés en rotation par rapport à la pièce de référence 306, C₁.

Les moyens de transformation du déplacement des écrous de liaison en un déplacement des pièces de transport 305, C₂ sont formés par une liaison rigide entre ces écrous de liaison et les pièces de transport, par exemple par montage à force des extrémités 351 des tiges 305 dans une ouverture des palets 308.

La première butée axiale C₁₁ et la deuxième butée axiale C₁₂ de chaque tige 302 filetée sont formées par l'écrou 311 et la croix 310 de Malte, placés contre les appuis A₁₁ et A₁₂ formés respectivement par la réservation 368 et la paroi 367 du boîtier C₁, 306.

Le dispositif 304 à intermittence permet avantageusement d'introduire un rapport de réduction entre l'arbre 303 de commande et les tiges 302 filetées. Pour un tour complet de l'arbre 303 de commande, les tiges 302 filetées n'effectuent qu'une portion de tour, dont la valeur dépend du nombre d'encoches de la croix 310 de Malte.

La tige 30 de distraction avec deux tiges 305 mobiles fonctionne comme suit.

La partie 352 extrême distale hors du boîtier 306 d'une première tige 305 mobile est fixée à une partie d'un os d'un membre d'un patient ou à un os. La partie 352 extrême distale hors du boîtier 306 de la deuxième tige 305 mobile est fixée à son tour à une autre partie de l'os, ou un autre os.

Le membre du patient est ensuite placé dans un champ magnétique tournant, sous l'effet duquel l'aimant entraine l'arbre 303 de commande en rotation. Un premier demi-tour de l'arbre 303 de commande provoque le déplacement d'une tige 305 mobile, puis le deuxième demi-tour provoque le déplacement de la deuxième tige 305 mobile.

Cette disposition permet que la totalité du couple de l'arbre 303 de commande s'applique sur chacune des deux tiges 302 filetées, chacune à leur tour.

Les tiges filetées 302 travaillent exclusivement en traction, qu'elles travaillent pour comprimer ou pour distracter le rachis ou le thorax.

Particulièrement quand la recherche d'un encombrement minimum du dispositif a conduit à un dispositif apte seulement à produire une faible force de distraction ou de compression, il est avantageux de laisser un jeu en translation soit entre la tige 302 filetée et le boîtier C₁, 306 soit entre ladite pièce taraudée qui coopère avec la tige 302 filetée et le palet 308. Ainsi, il sera possible de faciliter l'allongement de la tige de distraction, en manipulant le patient extérieurement, par exemple en le saisissant sous les épaules pour étirer manuellement son rachis en même temps qu'un couple sera appliqué sur l'aimant, puis en relâchant le patient et ainsi de suite. A chaque étirement, du fait du jeu en translation, la tige filetée sera momentanément sans charge et tournera donc spontanément sous l'effet du couple appliqué à l'aimant, déplaçant le palet 308 interdisant ainsi à la tige 305 mobile de reprendre sa place initiale quand l'étirement sera relâché. Dans cette mise en pratique, la tige 30 ne produit plus la distraction ou la compression, mais maintient celle obtenue par une manipulation externe.

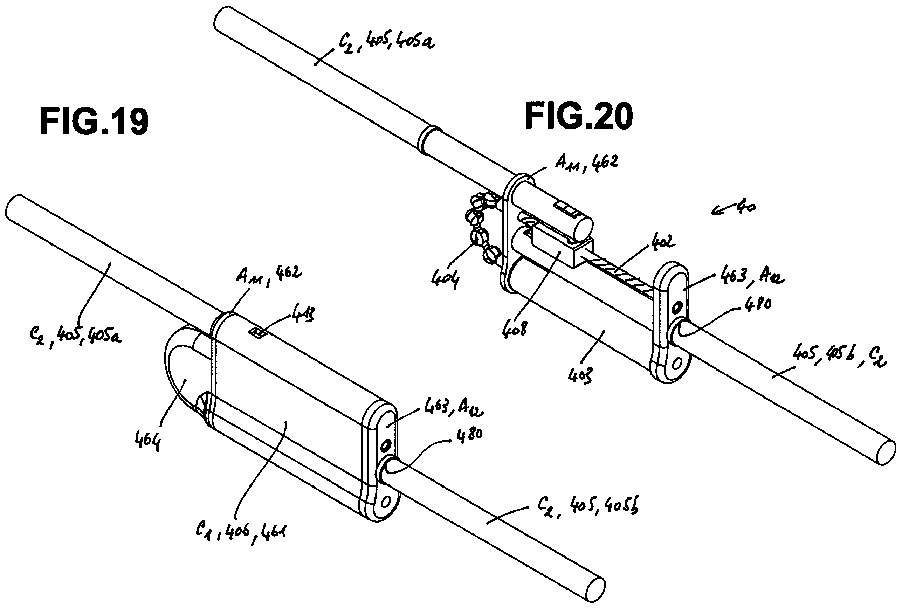

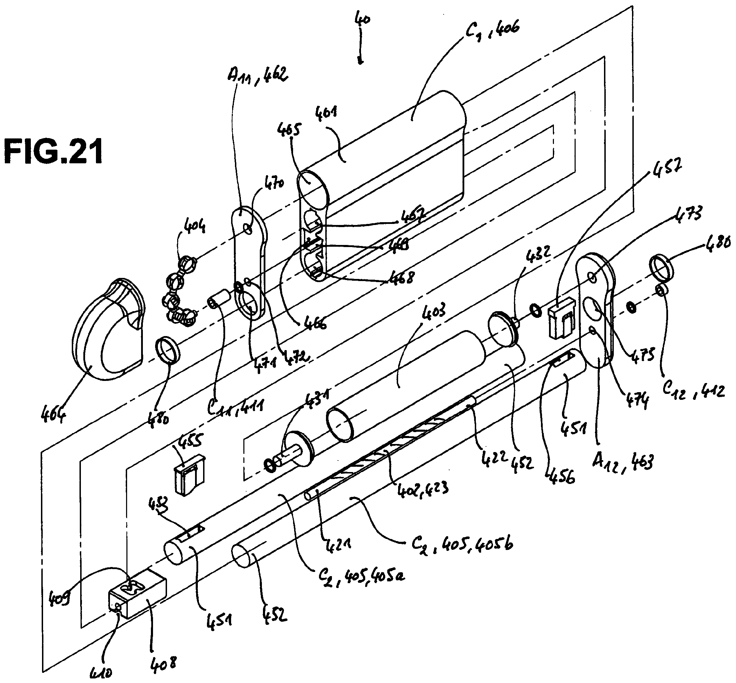

On se reporte maintenant aux figures 19 à 26 représentant un quatrième mode de réalisation.

Le distracteur 40 représenté en figures 16 à 26 comprend une tige 402 filetée, un arbre de commande 403, un joint de transmission 404 entre la tige 402 filetée et l'arbre de commande 403.

Le joint de transmission 404 est par exemple formé par une chaîne de joints de cardans.

Le distracteur 40 comporte deux tiges mobiles 405.

La tige filetée 402 et l'arbre 403 de commande ne sont pas coaxiaux. Dans le mode de réalisation représenté, l'axe de rotation de la tige filetée 402 est sensiblement parallèle à l'axe de rotation de l'arbre de commande 403. Les tiges mobiles 405 sont montées coulissantes, à l'intérieur d'un boîtier 406, les axes de coulissement de ces tiges mobiles 405 étant sensiblement disposés dans le plan contenant les axes de rotation de la tige filetée 402 et de l'arbre de commande 403.

Dans le mode de réalisation représenté, la tige filetée 402 s'étend entre et sensiblement à mi distance des axes de coulissement des tiges mobiles 405.

La tige filetée 402 comporte deux parties 421, 422 extrêmes lisses de part et d'autre d'une portion 423 filetée.

L'arbre de commande 403 se présente sous la forme d'un cylindre creux, dans lequel est fixé un aimant, par exemple au moyen d'une colle à base de silicone. A chacune de ses extrémités, l'arbre de commande comporte un ergot axial 431, 432.

Les tiges mobiles 405 comportent chacune une partie extrême proximale et une partie extrême distale. Les termes « distal », « proximal » sont employés ici en référence au boîtier 406.

La partie extrême distale 452 de chaque tige mobile 405 est apte à recevoir des moyens de liaison (non représentés), tels que des crochets, des vis ou des rubans, aux os (en particulier vertèbres, côtes ou bassin) auxquels on souhaite la fixer.

Dans le mode de réalisation représenté, les tiges 405 mobiles sont sensiblement droites et parallèles et peuvent être recoupées et courbées par le chirurgien. Dans d'autres modes de réalisation, non représentés, au moins l'une de ces tiges 405 mobiles peut être fournie cintrée.

Ainsi qu'il apparaît sur les figures, les deux tiges 405 mobiles s'étendent sensiblement symétriquement de part et d'autre de la tige filetée 402.

Un écrou de liaison 408 est monté sur la tige filetée 402 en étant guidé en rotation par rapport à la pièce de référence C₁, 406. Cet écrou de liaison 408 assure la liaison entre les pièces de transport C₂, 405 et la tige filetée 402. Cet écrou de liaison 408 est pourvu de deux réservations 409, 410 à ouvertures opposées.

Le boîtier 406 est formé d'une coque principale 461, de deux flasques 462, 463 et d'une coque 464.

La coque principale 461 comprend un premier conduit 465 sensiblement cylindrique, logeant l'arbre de commande 403. La coque principale 461 comprend un deuxième conduit 466 à trois lumières. Une première tige 405a mobile est montée coulissante dans une première lumière 467. Une deuxième tige mobile 405b est montée coulissante dans une deuxième lumière 468. L'écrou de liaison 408 est monté coulissant dans une troisième lumière 469.

Le premier flasque 462 est pourvu d'une première ouverture 470 de passage d'un ergot 431 de l'arbre de commande 403. Cet ergot 431 traverse ainsi le premier flasque 462 et est assemblé au joint de transmission 404. Le premier flasque 462 est pourvu d'une deuxième ouverture 471, au travers de laquelle coulisse la deuxième pièce mobile 405b, suivant une direction F1. Le premier flasque 462 est en outre pourvu d'une troisième ouverture 472 de montage d'une partie extrême 421 de la tige filetée 402. Cette partie extrême 421 traverse le premier flasque 462 et est assemblé au joint de transmission 404.

Le deuxième flasque 463 est pourvu d'une première ouverture 473 de passage d'un ergot 432 de l'arbre de commande 403. Le deuxième flasque 463 est pourvu d'une deuxième ouverture 474 de montage d'une partie extrême 422 de la tige filetée 402. Le deuxième flasque 463 est, en outre, pourvu d'une troisième ouverture 475 au travers de laquelle coulisse la première pièce mobile 405a.

Un premier écrou 411 forme une première butée C₁₁ contre le premier flasque 462, ce premier flasque 462 formant un premier appui A₁₁ pour bloquer longitudinalement la tige 402 filetée.

Un deuxième écrou 412 forme une deuxième butée C₁₂ contre le deuxième flasque 463, ce deuxième flasque 463 formant un deuxième appui A₁₂ pour bloquer longitudinalement la tige 402 filetée.

La première pièce 405a mobile est pourvue, à sa partie extrême proximale 451, d'un trou traversant 453 de passage d'un premier mécanisme à cliquet 455.

La deuxième pièce 405b mobile est également pourvue, à sa partie extrême proximale 451, d'un trou traversant 456 de passage d'un deuxième mécanisme à cliquet 457.

Le premier mécanisme à cliquet 455 comprend un cliquet 458, mobile à l'encontre d'un moyen élastique tel que ressort, non représenté, ce cliquet étant pourvu d'une rampe 460 d'encliquetage

Le deuxième mécanisme à cliquet 457 comprend un cliquet 459, ce cliquet 459 étant mobile à l'encontre d'un moyen élastique tel que ressort, non représenté.

Sur chacune des deux pièces mobiles 405a, 405b, une bague 480 est avantageusement soudée, permettant de visualiser facilement le mouvement accompli, sur une radiographie.

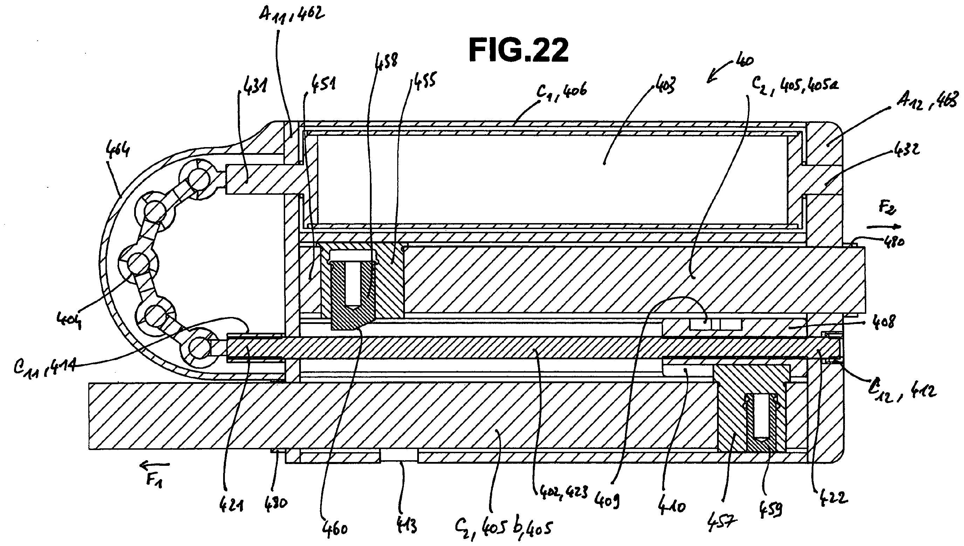

Dans une première position extrême, représentée en figure 22, le deuxième mécanisme à cliquet 457 est en butée dans une réservation 410 de l'écrou de liaison 408.

La rotation de l'arbre de commande 403 est transmise à la tige filetée 402 par le joint de transmission 404. La rotation de la tige filetée 402 provoque le déplacement de l'écrou de liaison 408.

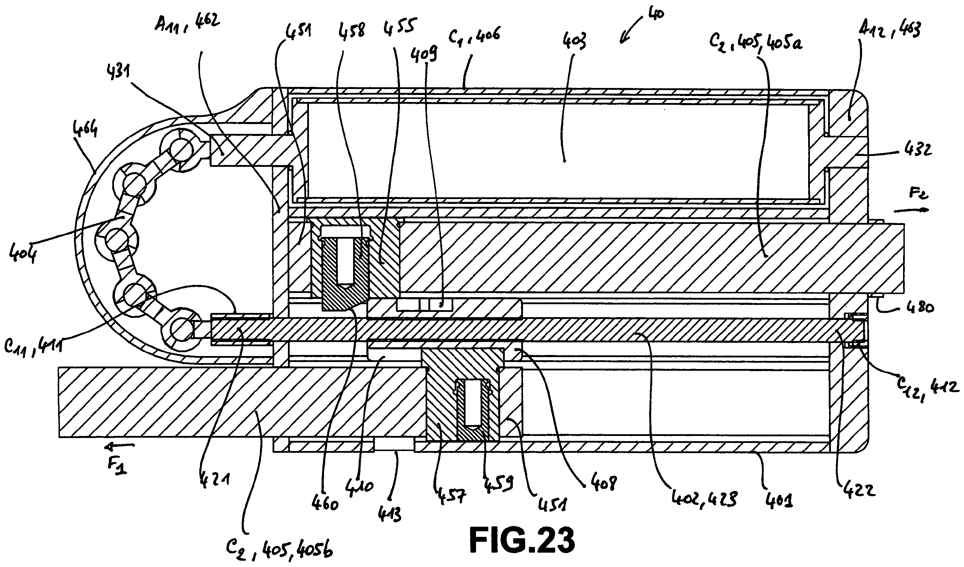

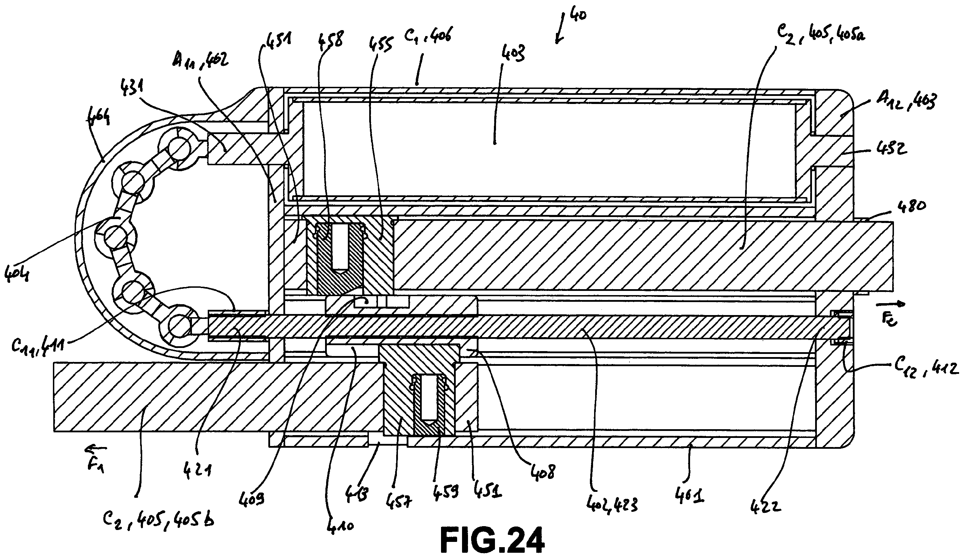

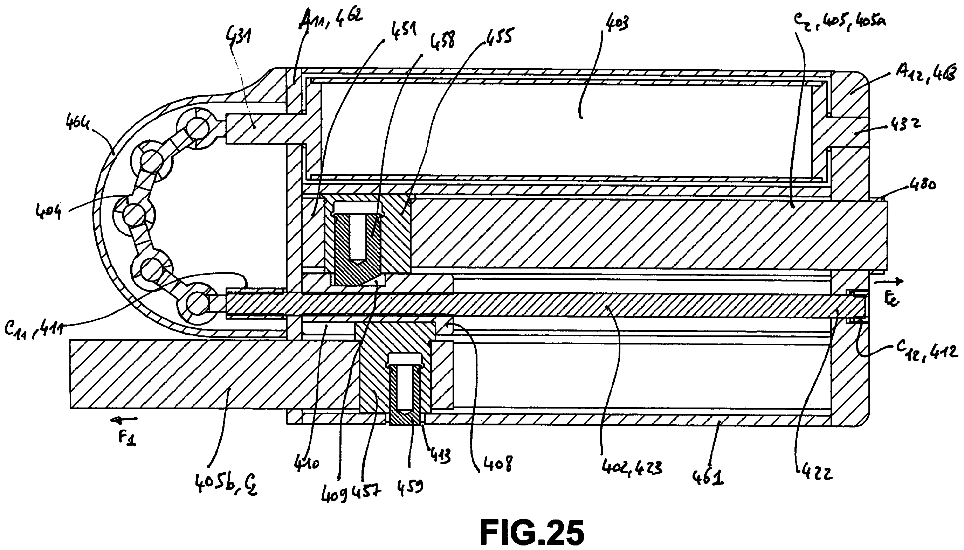

Le déplacement de l'écrou de liaison 408 entraîné le coulissement de sortie de la deuxième pièce mobile C₂, 405b, suivant la direction F1, depuis la position extrême rentrée de cette pièce de transport C₂, 405b, représentée en figure 22, jusqu'à sa position extrême sortie représentée en figure 25, deux positions intermédiaires étant représentées en figures 23 et 24.

Lorsque la deuxième pièce de transport C₂, 405b est en position extrême sortie, le cliquet 459 du deuxième mécanisme à cliquet 457 vient se loger dans une réservation, par exemple un trou traversant 413, de la pièce de référence C₁, 406. Cet état est représenté en figure 25. Le mouvement de sortie du cliquet 459 est par exemple provoqué par un ressort de compression, non représenté.

Avantageusement, lorsque le cliquet 459 associé à la deuxième pièce de transport 405b est en position sortie et bloqué par rapport à la pièce de référence C₁, 406, le cliquet 458 associé à la première pièce de transport 405a est logé dans la réservation 409 de l'écrou de liaison 408 et la première pièce de transport 405a est en position extrême rentrée. La rampe 460 d'encliquetage facilite l'entrée du cliquet 458 en butée dans la réservation 409 de l'écrou 408.

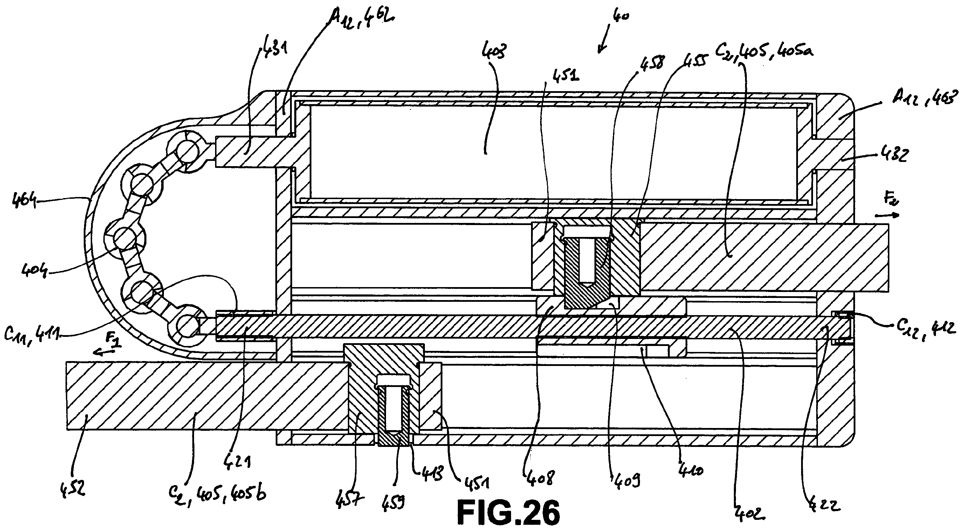

Ainsi, un mouvement de rotation de l'arbre de commande 403 provoque le mouvement de sortie de la première pièce de transport 405a, suivant la direction F2, depuis sa position extrême rentrée, représentée en figure 25, jusqu'à sa position extrême sortie (non représentée), une position intermédiaire étant représentée en figure 26. Lors du mouvement de sortie de la première pièce de transport 405a, la seconde pièce de transport 405b reste en position, par le verrouillage du cliquet 459 dans la réservation 413 du boîtier 406.

Le boîtier 406 forme une première pièce C₁, dite pièce de référence, les tiges 405a, 405b mobiles forment deux pièces C₂ de transport, les butées C₁₁ et C₁₂ de la tige 402 filetée sont formées par les écrous 411, 412 de butée axiale placés contre les appuis A₁₁ et A₁₂ formés respectivement par les flasques 462, 463 du boîtier C₁, 406.

Le moyen de liaison entre chaque pièce de transport 405a, 405b et la tige filetée 402 est formé par un écrou de liaison 408, monté sur la tige fileté 402 et guidé en rotation par rapport à la pièce de référence 406, C₁.

Les moyens de transformation du déplacement de cet écrou de liaison 408 en un déplacement de chaque pièce de transport 405a, 405b comprennent deux cliquets 458, 459, de structures avantageusement semblables.

Ce mode de réalisation préféré de l'invention offre un potentiel d'allongement important dans un volume particulièrement compact, et autorise en outre le contrôle successif des tiges 405a, 405b mobiles.

Dans les modes de réalisation décrits, la commande est réalisée grâce à un aimant intégré au dispositif. Avantageusement, cet aimant est un aimant néodyme ayant une température admissible en fonctionnement d'environ 150°C, pour permettre la stérilisation dudit dispositif par tous moyens et notamment à la vapeur surchauffée à 134°C sans risque de détérioration dudit aimant permanent.

Une rotation de l'aimant peut être facilement obtenue en approchant un dipôle magnétique de l'aimant, lequel agit au travers des tissus corporels du patient. Lorsque le dipôle a effectué un tour complet, l'aimant aura également effectué un tour complet. Ainsi, aucune partie du dispositif ne fait saillie hors du corps du patient.

Des trains d'engrenage peuvent être interposés entre l'arbre de commande et l'arbre de transmission, et/ou entre l'arbre de transmission et la tige filetée, afin de réaliser des rapports de réduction entre la commande et la tige filetée. Dans le cas où un seul aimant commande deux tiges mobiles, des rapports de réduction différents peuvent avantageusement permettre des déplacements distincts, bien que proportionnels dans une direction et une autre.

Les dispositifs présentés permettent de réaliser le déplacement de tissus, dans le sens d'une distraction ou d'une compression ou en alternant l'un et l'autre, mettant en oeuvre un nombre restreint de pièces.

L'utilisation d'un arbre de transmission flexible permettant de s'affranchir de l'alignement de l'arbre de commande avec la tige filetée, la forme générale du dispositif peut s'adapter au mieux à la forme des tissus dans lesquels il est implanté.

L'arbre flexible permet, en outre, de disposer d'un dispositif à double sens s'il est solidarisé à ses deux extrémités, ou bien d'utiliser une de ses extrémités comme un ressort de friction qui permettra ainsi la rotation de la tige filetée dans un sens, celui qui tend à serrer le ressort, mais pas dans le sens opposé dans lequel le ressort patinera.

Eventuellement, un système complémentaire de blocage de la rotation dans un sens peut être introduit, par exemple au moyen de ressorts ou de roulements. Des exemples de tels systèmes de blocage sont donnés dans les documents US 5 505 733 et WO 2004/019796.

L'utilisation de la tige filetée 2, 202, 302, 402 en traction permet d'éliminer l'influence du flambage sur le dimensionnement de la tige. Il est donc possible de réduire le diamètre de la tige filetée, par rapport aux dispositifs travaillant en compression.

Le dispositif suivant l'invention est avantageusement réalisé dans des matériaux résistants mécaniquement et bien tolérés par l'organisme, tels que des aciers inoxydables comme le 316L, des alliages de titane, des polymères tels que le Poly-Ether-Ether-Cétone (PEEK) ou, de préférence, comme des alliages hautes performances à base de chrome et de cobalt comme par exemple l'alliage austénitique commercialisé par la société Arcelor Mittal sous l'appellation PHYNOX (désignation AFNOR : K13C20N16Fe15D07) ou de Nickel et Cobalt tel le VALLOY-120 spécifique au procédé EFAB.

Par ailleurs, les surfaces soumises aux frottements dudit dispositif, en particulier la tige filetée, peuvent avantageusement recevoir un traitement de surface anti-usure et/ou diminuant leur coefficient de frottement à base de carbone amorphe diamantin ou de bisulfure de tungstène, par exemple.

Ainsi, la combinaison du faible diamètre de la tige filetée, permis par son travail en traction, et du bas coefficient de friction obtenu grâce auxdits traitements de surface permet l'entraînement de la tige filetée par un couple modeste au regard de la charge appliquée, et l'utilisation de moyens simples pour la création de ce couple, tels qu'une transmission par aimants permanents d'efforts appliqués directement à la main.

Le diamètre de la tige filetée 2, 202, 302, 402 est couramment compris entre un et trois millimètres et ne dépasse pas quatre millimètres, pour une prothèse de patient adulte par exemple.

Le dispositif selon l'invention peut avantageusement être réalisé sans assemblage , par exemple par la technique EFAB, proposée par la société Microfabrica, à l'exception d'une première trappe dans la pièce de référence et d'une seconde trappe dans la structure qui entoure l'aimant, trappes qui permettent l'insertion et le collage de l'aimant. Ce mode de réalisation sera particulièrement avantageux pour les applications maxillo-faciales, cardiaques et à la chirurgie de la main du dispositif selon l'invention, qui nécessitent une extrême compacité.

### Possibilités d'application industrielle

Le dispositif suivant l'invention est particulièrement utile notamment pour la réalisation de tiges pour la correction du rachis ou du thorax, de clous et de plaques d'allongement ou de transport osseux et de prothèses à croissance.

Les dispositifs selon l'invention trouvent également application dans l'allongement ou l'extension ou la déformation des tissus mous tels qu'une partie de l'intestin, ou bien encore pour le cerclage artériel, les anneaux de valvuloplastie à géométrie évolutive, les anneaux gastriques.

## Revendications

1. Dispositif de déplacement de tissus à l'intérieur de l'organisme, notamment de tissus osseux, ce dispositif comprenant :
- une première pièce (6, 206, 306, 406), dite pièce de référence (C₁);
- une seconde pièce (53, 205, 305, 405), dite pièce de transport (C₂), montée coulissante par rapport à la pièce de référence (C₁) ;
- une tige (2, 202, 302, 402) comportant au moins un filetage, dite tige filetée, montée pivotante par rapport à la pièce de référence (C₁) ;
- un arbre (3, 203, 303, 403) de commande ;
- des moyens d'entraînement (4, 204, 304, 404) reliant l'arbre de commande à la tige filetée,
- un moyen de liaison, dit écrou de liaison (5, 208, 308, 408), entre la pièce de transport (C₂) et la tige filetée, cet écrou de liaison étant monté sur la tige filetée et étant guidé en rotation par rapport à la pièce de référence (C₁);
- des moyens de transformation du déplacement de l'écrou de liaison le long de la tige filetée en un déplacement de la pièce de transport (C₂) par rapport à la pièce de référence (C₁),
ce dispositif étant **caractérisé en ce que**, pour limiter la translation longitudinale de ladite tige filetée par rapport à la pièce de référence (C₁), une première butée (C₁₁) et une seconde butée (C₁₂) solidaires de la tige filetée coopèrent respectivement avec un premier appui (A₁₁) et un second appui (A₁₂), ces appuis étant solidaires de la pièce de référence (C₁) et étant placés à distance l'un de l'autre entre lesdites butées (C₁₁, C₁₂), l'écrou de liaison étant apte à se déplacer le long de la tige filetée entre le premier appui (A₁₁) et le second appui (A₁₂).

2. Dispositif selon la première revendication, **caractérisé en ce que** les moyens d'entraînement comportent au moins un ressort hélicoïdal.

3. Dispositif selon les revendications 1 ou 2, **caractérisé en ce que** les moyens d'entraînement comportent un dispositif à intermittence (304) solidaire de la tige filetée (302) et entraîné en mouvement par l'arbre de commande (303), ce dispositif à intermittence (304) étant apte à transformer un mouvement de rotation continue de l'arbre de commande (303) en un mouvement intermittent de la tige filetée (302).

4. Dispositif selon la revendication 3, **caractérisé en ce que** les moyens d'entraînement comportent une croix de Malte (310) solidaire de la tige filetée (302).

5. Dispositif selon l'une quelconque des revendications 1 à 4, **caractérisé en ce que** les moyens de transformation comportent une liaison rigide entre l'écrou de liaison et la pièce de transport.

6. Dispositif suivant la revendication 5, **caractérisé en ce que** le guidage en rotation de l'écrou de liaison par rapport à la pièce de référence est un guidage hélicoïdal tel que ledit écrou de liaison tourne d'un angle compris entre 10 et 180° quand il se déplace dudit premier (A₁₁) audit second appui ou vice-versa (A₁₂)

7. Dispositif selon l'une quelconque des revendications précédentes, **caractérisé en ce qu'**il comprend deux pièces de transport (305) et deux tiges filetées (302).

8. Dispositif selon la revendication 7, **caractérisé en ce que** les filetages des deux tiges filetées ont des caractéristiques de diamètre, de sens, ou de pas différentes.

9. Dispositif selon l'une quelconque des revendications précédentes, **caractérisé en ce que** le diamètre de la (ou des) tige(s) filetée(s) (2, 202, 302, 402) est inférieur à 4 mm.

10. Dispositif selon l'une quelconque des revendications 7 à 9, **caractérisé en ce que** les pièces de transport (305, C₂) sont commandées par un arbre de commande commun (303).

11. Dispositif selon l'une quelconque des revendications précédentes, **caractérisé en ce qu'**au moins une pièce de transport (205, 305, 405) est pourvue de moyens de liaisons aux os, tels que crochets, vis ou rubans.

12. Dispositif selon l'une quelconque des revendications précédentes, **caractérisé en ce que** l'arbre de commande (3, 203, 303, 403) comprend un aimant permanent dont la direction de magnétisation est sensiblement perpendiculaire à l'axe de rotation dudit arbre de commande.

13. Dispositif selon l'une quelconque des revendications précédentes, **caractérisé en ce que** la tige filetée (2) a son axe de rotation sensiblement parallèle et distant à l'axe de rotation de l'arbre de commande (3)et la pièce de référence (C₁) comprend un fourreau (6) dans lequel coulisse un chariot (5) de transport osseux sur lequel une pièce de transport (53) est montée, et le chariot (5) de transport osseux est pourvu d'un perçage (51) longitudinal taraudé traversant, distant de l'axe du fourreau (6), ce perçage étant complémentaire du filetage de la tige filetée (2).

14. Dispositif selon la revendication 13, **caractérisé en ce que** le chariot (5) de transport osseux est pourvu d'un perçage diamétral (52) logeant une vis à os (53) formant pièce de transport, cette vis à os (53) étant montée coulissante dans deux gorges (61) parallèles longitudinales du fourreau (6).

15. Dispositif selon l'une quelconque des revendications 1 à 12 ou selon la revendication 14, **caractérisé en ce qu'**il comprend une seule tige (402) filetée, les moyens de transformation étant aptes à transmettre un déplacement de l'écrou de liaison (408) dans un premier sens de déplacement, en un coulissement d'une première pièce de transport (405a, C₂) par rapport à la pièce de référence (406, C₁) jusque vers une position extrême, le dispositif comprenant des moyens de verrouillage de la première pièce de transport (405a, C₂) en cette position extrême.

16. Dispositif selon la revendication 15, **caractérisé en ce que** les moyens de transformation sont aptes à transmettre un déplacement de l'écrou de liaison (408) dans un deuxième sens de déplacement opposé audit premier sens, en un coulissement d'une deuxième pièce de transport (405b, C₂) par rapport à la pièce de référence (406, C₁).

## Patentansprüche

1. Vorrichtung zum Verschieben von Gewebe im Inneren des Organismus, insbesondere von Knochengewebe, wobei diese Vorrichtung Folgendes umfasst:
- ein erstes Teil (6, 206, 306, 406), das so genannte Bezugsteil (C₁),
- ein zweites Teil (53, 205, 305, 405), das so genannte Transportteil (C₂), das bezüglich des Bezugsteils (C₁) gleitend montiert ist,
- eine mindestens ein Gewinde aufweisende Stange (2, 202, 302, 402), die so genannte Außengewindestange, die bezüglich des Bezugsteils (C₁) schwenkend montiert ist,
- eine Steuerwelle (3, 203, 303, 403),
- Antriebsmittel (4, 204, 304, 404), die die Steuerwelle mit der Außengewindestange verbinden,
- ein Verbindungsmittel, die so genannte Verbindungsmutter (5, 208, 308, 408) zwischen dem Transportteil (C₂) und der Außengewindestange, wobei diese Verbindungsmutter an der Außengewindestange montiert ist und bezüglich des Bezugsteils (C₁) drehgeführt ist,
- Mittel zur Umwandlung der Verschiebung der Verbindungsmutter entlang der Außengewindestange in eine Verschiebung des Transportteils (C₂) bezüglich des Bezugsteils (C₁),
**dadurch gekennzeichnet, dass** ein erster Anschlag (C₁₁) und ein zweiter Anschlag (C₁₂), die fest mit der Außengewindestange verbunden sind, mit einem ersten Auflager (A₁₁) bzw. einem zweiten Auflager (A₁₂) zusammenwirken, um die Längstranslation der Außengewindestange bezüglich des Bezugsteils (C₁) zu begrenzen, wobei diese Auflager fest mit dem Bezugsteil (C₁) verbunden und zwischen den Anschlägen (C₁₁, C₁₂) voneinander beabstandet platziert sind, wobei die Verbindungsmutter geeignet ist, sich entlang der Außengewindestange zwischen dem ersten Auflager (C₁₁) und dem zweiten Auflager (C₁₂) zu verschieben.

2. Vorrichtung nach dem ersten Anspruch, **dadurch gekennzeichnet, dass** die Antriebsmittel mindestens eine Spiralfeder aufweisen.

3. Vorrichtung nach Anspruch 1 oder 2, **dadurch gekennzeichnet, dass** die Antriebsmittel eine fest mit der Außengewindestange (302) verbundene und durch die Steuerwelle (303) bewegungsangetriebene Taktvorrichtung (304) aufweisen, wobei diese Taktvorrichtung (304) geeignet ist, eine kontinuierliche Drehbewegung der Steuerwelle (303) in eine intermittierende Bewegung der Außengewindestange (302) umzuwandeln.

4. Vorrichtung nach Anspruch 3, **dadurch gekennzeichnet, dass** die Antriebsmittel ein fest mit der Außengewindestange (302) verbundenes Malteserkreuz (310) aufweist.

5. Vorrichtung nach einem der Ansprüche 1 bis 4, **dadurch gekennzeichnet, dass** die Umwandlungsmittel eine starre Verbindung zwischen der Verbindungsmutter und dem Transportteil aufweisen.

6. Vorrichtung nach Anspruch 5, **dadurch gekennzeichnet, dass** die Drehführung der Verbindungsmutter bezüglich des Bezugsteils eine spiralförmige Führung ist, so dass sich die Verbindungsmutter in einem Winkel zwischen 10 und 180° dreht, wenn sie sich von dem ersten (A₁₁) zum zweiten Auflager (A₁₂) oder umgekehrt verschiebt.

7. Vorrichtung nach einem der vorhergehenden Ansprüche, **dadurch gekennzeichnet, dass** sie zwei Transportteile (305) und zwei Außengewindestangen (302) umfasst.

8. Vorrichtung nach Anspruch 7, **dadurch gekennzeichnet, dass** die Gewinde der beiden Außengewindestangen unterschiedliche Durchmesser-, Richtungs- oder Steigungscharakteristiken haben.

9. Vorrichtung nach einem der vorhergehenden Ansprüche, **dadurch gekennzeichnet, dass** der Durchmesser der Außengewindestange(n) (2, 202, 302, 402) kleiner als 4 mm ist.

10. Vorrichtung nach einem der Ansprüche 7 bis 9, **dadurch gekennzeichnet, dass** die Transportteile (305, C₂) durch eine gemeinsame Steuerwelle (303) gesteuert werden.

11. Vorrichtung nach einem der vorhergehenden Ansprüche, **dadurch gekennzeichnet, dass** mindestens ein Transportteil (205, 305, 405) mit Knochenverbindungsmitteln wie Haken, Schrauben oder Bändern versehen ist.

12. Vorrichtung nach einem der vorhergehenden Ansprüche, **dadurch gekennzeichnet, dass** die Steuerwelle (3, 203, 303, 403) einen Permanentmagnet umfasst, dessen Magnetisierungsrichtung im Wesentlichen senkrecht zu der Rotationsachse der Steuerwelle verläuft.

13. Vorrichtung nach einem der vorhergehenden Ansprüche, **dadurch gekennzeichnet, dass** die Rotationsachse der Außengewindestange (2) im Wesentlichen parallel zu der Rotationsachse der Steuerwelle (3) und von ihr beabstandet verläuft und das Bezugsteil (C₁) eine Hülse (6) umfasst, in der ein Knochentransportschlitten (5) gleitet, auf dem ein Transportteil (53) montiert ist, und der Knochentransportschlitten (5) mit einer Längsdurchgangsbohrung (51) mit einem Innengewinde versehen ist, die von der Achse der Hülse (6) beabstandet ist, wobei diese Bohrung zum Außengewinde der Außengewindestange (2) komplementär ist.

14. Vorrichtung nach Anspruch 13, **dadurch gekennzeichnet, dass** der Knochentransportschlitten (5) mit einer diametralen Bohrung (52) versehen ist, in der eine Knochenschraube (53) aufgenommen ist, die das Transportteil bildet, wobei diese Knochenschraube (53) gleitend in zwei parallelen Längskehlen (61) der Hülse (6) montiert ist.

15. Vorrichtung nach einem der Ansprüche 1 bis 12 oder nach Anspruch 14, **dadurch gekennzeichnet, dass** sie eine einzige Außengewindestange (402) umfasst, wobei die Umwandlungsmittel geeignet sind, eine Verschiebung der Verbindungsmutter (408) in eine erste Verschiebungsrichtung in ein Gleiten eines ersten Transportteils (405a, C₂) bezüglich des Bezugsteils (406, C₁) bis zu einer Extremposition zu übertragen, wobei die Vorrichtung Mittel zur Verriegelung des ersten Transportteils (405a, C₂) in dieser Extremposition umfasst.

16. Vorrichtung nach Anspruch 15, **dadurch gekennzeichnet, dass** die Umwandlungsmittel geeignet sind, eine Verschiebung der Verbindungsmutter (408) in eine der ersten Richtung entgegengesetzte zweite Verschiebungsrichtung in ein Gleiten eines zweiten Transportteils (405b, C₂) bezüglich des Bezugsteils (406, C₁) zu übertragen.

## Claims

1. Device for moving tissue inside the body, in particular bone tissue, said device including:
- a first part **(6, 206, 306, 406)** referred to as the reference part (**C₁**);
- a second part **(53, 205, 305, 405),** referred to as the transport part (**C₂**), which is slidably mounted relative to the reference part (**C₁**);
- a rod **(2, 202, 302, 402)** comprising at least one thread, referred to as the threaded rod, pivotably mounted relative to the reference part (**C₁**);
- a control shaft **(3, 203, 303, 403);**
- driving means **(4, 204, 304, 404**) connecting the control shaft to the threaded rod;
- connecting means, referred to as the connecting nut **(5, 208, 308, 408),** between the transport part (**C₂**) and the threaded rod, said connecting nut being mounted onto the threaded rod and being rotatably guided relative to the reference part (**C₁**);
- means for converting the movement of the connecting nut along the threaded rod into a movement of the transport part (**C₂**) relative to the reference part (**C₁**),
said device being **characterized in that**, in order to limit the longitudinal translation of said threaded rod relative to the reference part (**C₁**), a first abutment (**C₁₁**) and a second abutment (**C₁₂**) rigidly connected to the threaded rod respectively cooperate with a first bearing (**A₁₁**) and a second bearing (**A₁₂**), these bearings being rigidly connected to the reference part (**C₁)** and being placed at a distance from one another between said abutments (**C₁₁**, **C₁₂**), the connecting nut being movable along the threaded rod between the first bearing (**A₁₁**) and second bearing (**A₁₂**).

2. Device according to the first claim, **characterized in that** the driving means comprise at least one helical spring.

3. Device according to Claims 1 or 2, **characterized in that** the driving means comprise an intermittent device **(304)** rigidly connected to the threaded rod **(302)** and moved by the control shaft **(303),** said intermittent device **(304)** being capable of converting a continuous rotational movement of the control shaft **(303)** into an intermittent movement of threaded rod **(302).**

4. Device according to Claim 3, **characterized in that** the driving means comprise a Geneva wheel mechanism **(310)** rigidly connected to the threaded rod **(302).**

5. Device according to any one of Claims 1 to 4, **characterized in that** the conversion means comprise a rigid connection between the connecting nut and the transport part.

6. Device according to Claim 5, **characterized in that** the rotational guiding of the connecting nut relative to the reference part is a helical guiding such that said connecting nut rotates by an angle comprised between 10 and 180° when it moves from said first (**A₁₁**) to said second (**A₁₂**) bearing, or vice versa.

7. Device according to any one of the preceding claims, **characterized in that** it comprises two transport parts **(305)** and two threaded rods **(302).**

8. Device according to Claim 7, **characterized in that** the threads of the two threaded rods have different diameter, direction, or pitch characteristics.

9. Device according to any one of the preceding claims, **characterized in that** the diameter of the threaded rod(s) **(2, 202, 302, 402)** is smaller than 4 mm.

10. Device according to any one of Claims 7 to 9, **characterized in that** the transport parts (**305**, **C₂**) are controlled by a shared control shaft (**303**).

11. Device according to any one of the preceding claims, **characterized in that** at least one transport part **(205, 305, 405)** is provided with connecting means for connecting to the bones, such as hooks, screws, or tapes.

12. Device according to any one of the preceding claims, **characterized in that** the control shaft **(3, 203, 303, 403)** comprises a permanent magnet whereof the direction of magnetization is substantially perpendicular to the axis of rotation of the said control shaft.

13. Device according to any one of the preceding claims, **characterized in that** the axis of rotation of the threaded rod **(2)** is substantially parallel to and at a distance from the axis of rotation of the control shaft (**3**) and the reference part (**C₁)** comprises a sheath **(6)** in which a bone transport carriage **(5)** slides on which a transport part **(53)** is mounted, and the bone transport carriage **(5)** is provided with a through tapped longitudinal piercing **(51),** at a distance from the axis of the sheath **(6),** said piercing being complementary to the threading of the threaded rod **(2).**

14. Device according to Claim 13, **characterized in that** the bone transport carriage **(5)** is provided with a diametric piercing **(52)** housing a bone screw **(53)** forming the transport part, said bone screw **(53)** being slidably mounted in two longitudinal parallel grooves **(61)** of the sheath **(6).**

15. Device according to any one of Claims 1 to 12 or according to Claim 14, **characterized in that** it comprises a single threaded rod **(402),** the conversion means being capable of transmitting a movement from the connecting nut **(408)** in a first direction of movement, by sliding a first transport part **(405a, C₂**) relative to the reference part **(406, C₁**) as far as an end position, the device comprising means for locking the first transport part **(405a, C₂**) in said end position.

16. Device according to Claim 15, **characterized in that** the conversion means are capable of transmitting a movement of the connecting nut **(408)** in a second direction of movement opposite the first direction, by sliding a second transport part **(405b, C₂**) relative to the reference part **(406, C₁**).
